(19) Europäisches Patentamt
European Patent Office
Office européen des brevets

(11) Publication number: **0 180 812 B1**

(12) **EUROPEAN PATENT SPECIFICATION**

(45) Date of publication of patent specification: **15.05.91**

(51) Int. Cl.[5]: **C07D 219/06**, C07D 401/14, A61K 31/435

(21) Application number: **85112985.8**

(22) Date of filing: **14.10.85**

The file contains technical information submitted after the application was filed and not included in this specification

(54) **3,6 Bis(substituted) acridine derivatives.**

(30) Priority: **09.11.84 US 669917**
**09.11.84 US 669916**

(43) Date of publication of application:
**14.05.86 Bulletin 86/20**

(45) Publication of the grant of the patent:
**15.05.91 Bulletin 91/20**

(84) Designated Contracting States:
**AT BE CH DE FR GB IT LI NL SE**

(56) References cited:
**EP-A- 0 012 139**
**EP-A- 0 031 407**

**JOURNAL OF MEDICINAL CHEMISTRY, vol.26, 1983, pages 1710-1715, American Chemical Society, Columbus, Ohio, US; R.B. ANGIER et al.: "Synthesis of 3,6-Bis(aminoalkoxy)acridines and their effect on the immune system"**

(73) Proprietor: **AMERICAN CYANAMID COMPANY**
**1937 West Main Street P.O. Box 60**
**Stamford Connecticut 06904-0060(US)**

(72) Inventor: **Child, Ralph Grassing**
**432 Erhardt Road**
**Pearl River, NY 10965(US)**
Inventor: **Fields, Thomas Lynn**
**62 Amory Avenue**
**Pearl River, NY 10965(US)**
Inventor: **Wilkinson, Raymond George**
**7 Surrey Lane**
**Montvale, NJ 07645(US)**
Inventor: **Lin, Yang-I**
**4 Pelham Court**
**Nanuet, NY 10954(US)**

(74) Representative: **Wächtershäuser, Günter, Dr.**
**Tal 29**
**W-8000 München 2(DE)**

**EP 0 180 812 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

This invention relates to novel compounds of the following formula:

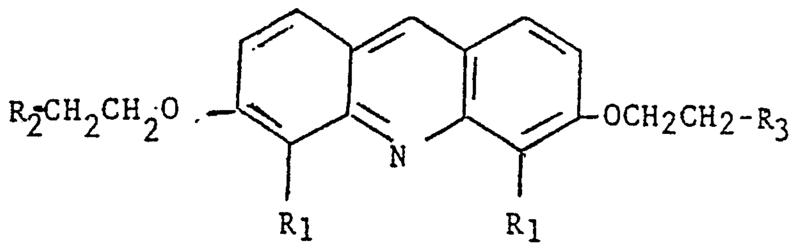

wherein $R_1$ is formyl, hydroxymethyl, aminomethyl, the group:

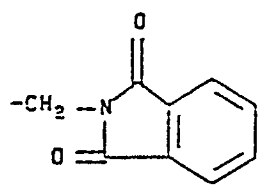

,

hydrogen, halogen, -COOH, and alkyl($C_1$-$C_3$), $R_2$ and $R_3$ may be the same or different and are piperidinyl, -N[alkyl($C_1$-$c_5$)]$_2$, pyrrolidino, morpholino, or the groups:

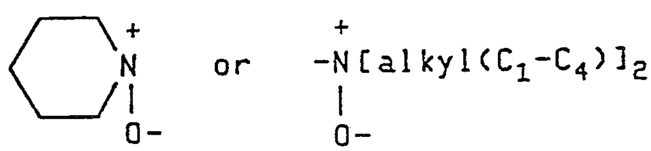

with the provisos that where $R_1$ is aminomethyl or

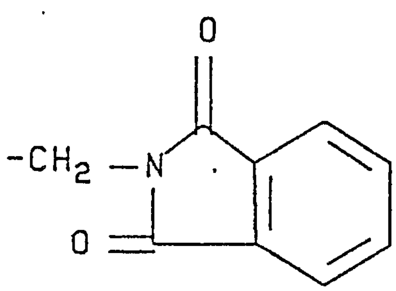

,

$R_2$ and $R_3$ must be pyrrolidino, morpholino, piperidinyl, or N-[alkyl($C_1$-$C_5$)]$_2$; and where $R_1$ is halogen, -COOH or alkyl($C_1$-$C_3$) then $R_7$ and $R_3$ must be piperidinyl or

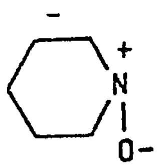,

and where R1 is hydrogen, R2 and R3 may be the same or different and are piperidinyl, piperidinyl-N-oxide, $-N(C_2H_5)_2$ or

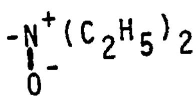

with the proviso that at least one of $R_2$ and $R_3$ must be in the N-oxide form; and the pharmaceutically acceptable salts thereof.

Preferred embodiments of the invention are as defined in Claims 2 to 6. Further embraced by the invention is a process for the preparation of the novel compounds as defined in Claims 7 to 11.

The compounds of the present invention can be used in a method of treating the immune response system in a warm-blooded animal which comprises administering to said animal an effective amount of a compound selected from those of the above formulas in association with a pharmaceutically acceptable carrier, adjuvant or diluent.

The use of immunomodulants and chemotherapeutic adjuvants constitutes a new therapeutic approach to the treatment of immune deficiencies and cancer and is based on the concept that there are distinctive antigens in or on most tumor cells (embryonal or transplantation antigens) that distinguish them from normal host cells. A majority of tumor immunologists favor the view that potentially malignant cells constantly arise but, because of their "foreigness", are normally eliminated by a competent humoral and cellular immune system. occasionally, however, tumor cells escape this immune surveillance and continue to reproduce and cancer results. The reason for the failure of the normally efficient immune surveillance mechanisms is not fully understood but it is thought that the immune system becomes less effective with increasing age. It is depressed in certain genetic immuno-deficiency diseases, in various bacterial, fungal or viral infections and in patients undergoing immuno-suppressive therapy. The growth of the neoplasm itself, as well as the various therapeutic modalities designed to treat the disease, e.g., cytotoxic chemotherapy and radiation, leads to a still greater depression of host resistance and results in an increased susceptibility to both exogenous and endogenous infections and perhaps accounts for the re-initiation of tumor growth and metastasis which frequently follows treatment-induced tumor remission.

If depression of the immune system can result in the growth of malignancies, regulation of any facet of the immune response may help the host to eliminate residual. cancer cells. Therefore, it is desirable to search for chemical agents (i.e., immunoregulants) capable of restoring and stimulating host immune defense mechanisms in order to overcome the deficiencies which account for susceptibility to disease and failure to eradicate the cancer. Such immunoregulating agents would likely be incapable of arresting the growth of a large tumor but their clinical utility would derive from their capacity to enhance normal immune surveillance mechanisms in patients whose tumor burden has been reduced by surgical, radiotherapeutic or chemotherapeutic methods.

Experimental studies in animals have demonstrated the antitumor potential of a number of immunoregulants including live organisms of bacillus Calmette-Guerin (BCG), heat-killed cells of Corynebacterium parvum, polynucleotides and the anthelmintic drug, levamisole. These substances have been shown to stimulate cellular immunity and to produce tumor regression. Some successes have been claimed in early clinical trials with BCG against malignant melanoma and acute leukemia and with levamisole against lung cancer and breast cancer. Although the anti-tumor effects produced by these agents have been promising, significant therapeutic benefits have yet to be realized. Since this is a new therapeutic approach, new drugs and methods of treatment must receive careful clinical evaluation in order to reveal their full potential.

Modern research is directed to the discovery of a drug similar to, but more potent than, known immunoregulants such as levamisole that would be effective in the eradication of tumor cells when used in conjunction with standard therapeutic measures. Stimulators of host resistance may be detected in animal models that can, in fact, detect both immunostimulators and anticancer agents. Mice are put in a condition simulating immunodepression common to cancer patients. This is accomplished by infecting mice with a

leukemia virus which produces both leukemia and a disease-related immunodepression. Effective drugs are recognized by their ability to restore or enhance the antibody response in the experimental mice, or to inhibit tumor progression.

Certain synthetic and naturally derived compounds have the ability to induce high levels of circulating interferon. Among these are bacterial endotoxins, intact bacteria and viruses, transplantable tumor cells and a variety of high and low molecular weight synthetic compounds such as poly I:C, tilorone and pyran copolymer [W. E. Stewart, The Interferon System, Springer-Verlag, Wien, New York(1979)]. Interferon has a major regulatory function in modulating cellular and humoral immune responses. Interferon, and inducers of interferon, "activate" macrophages to destroy tumor and virus infected cells, stimulate populations of immune cells to secrete lymphokines, protect against lethal infection by viruses and some bacterial species and stimulate the level of natural killer lymphocyte (NK-cell) activity in animals [Herberman, R. B. and Holden, H. T., Natural Cell-Mediated Immunity, Adv. Cancer Res., 27, 305-377 (1978) and Natural Killer Cells as Antitumor Effector Cells, J. Nat. Cancer Inst., 62 (3), 441-445 (1979)]. NK-cells play a major roll in immune surveillance in that they mediate the destruction of virus infected cells and a wide variety of syngeneic, allogenic and xenogeneic tumor cells when tested in vitro (Herberman, R. B. and Holden, N. T. vide supra). The role of NK-cells in protecting animals against virus infection appears certain. European Patent No. 0 012 139 to Murdock et al. discloses 3,6-bis(dialkylaminoalkoxy)acridines that are useful as immunostimulators. The compounds showed an ability to restore or enhance the antibody response in experimental mice, or to inhibit tumor progression. The compounds of the present invention are acridine derivatives with novel substituents at the 3,6 or 4,5 positions of the acridine nucleus useful as immunomodulators.

The compounds of this invention may be prepared in accordance with the following flowcharts and descriptions.

FLOWCHART A

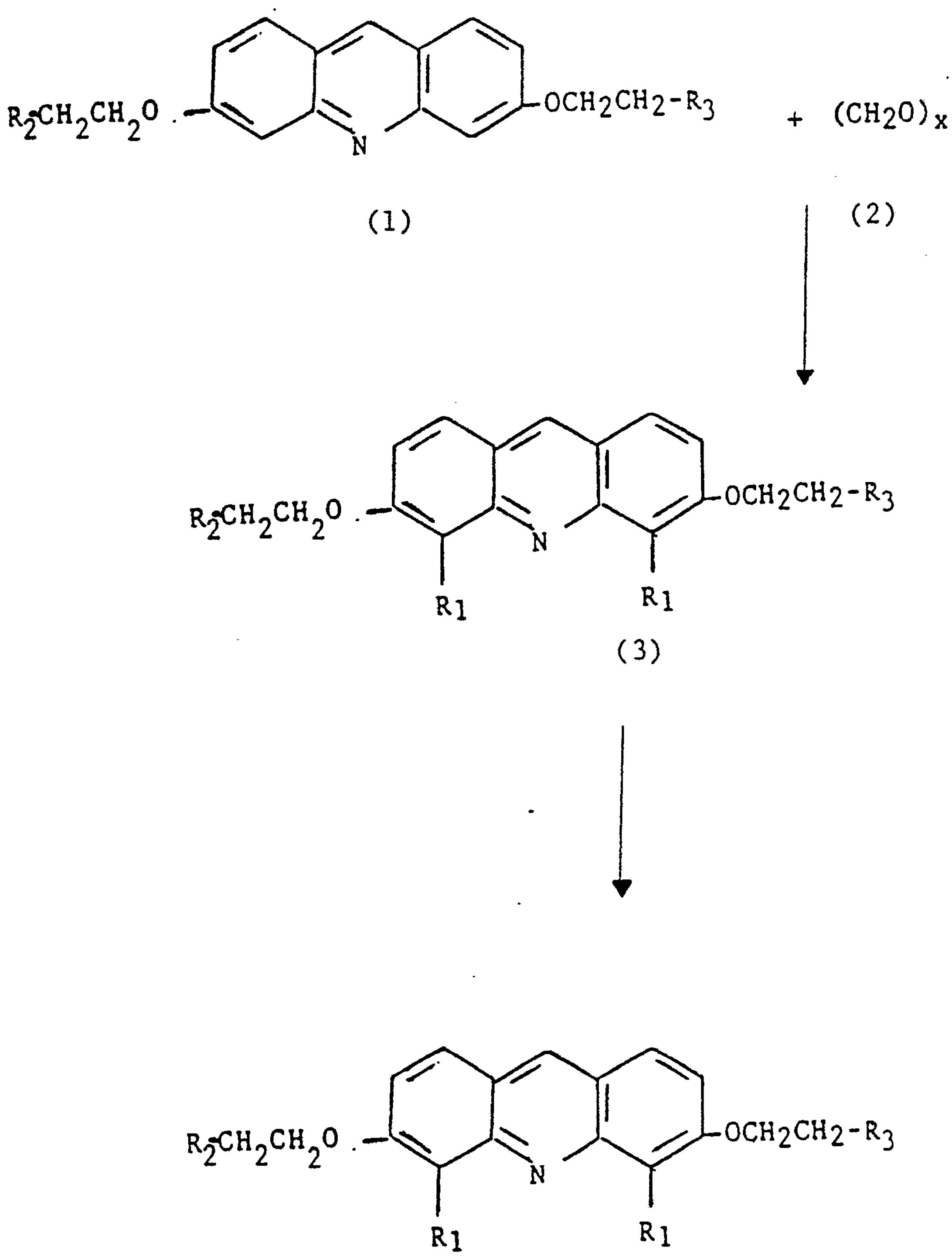

(1) (2) (3) (4)

In accordance with Flowchart A, a 3,6-bis(substituted)acridine (1), where $R_2$ and $R_3$ are as described above, in concentrated sulfuric acid is reacted with paraformaldehyde (2), where x is an integer greater than 1, for about 1/2 to about one hour then precipitated in ether, giving (3), where $R_1$ is $-CH_2OH$. The resulting solid is dissolved in dimethyl sulfoxide, triethylamine is added and the mixture reacted with sulfur trioxide-pyridine complex for about 1 to about 4 hours giving (4) where $R_2$ and $R_3$ are as described above and $R_1$ is formyl.

FLOWCHART B

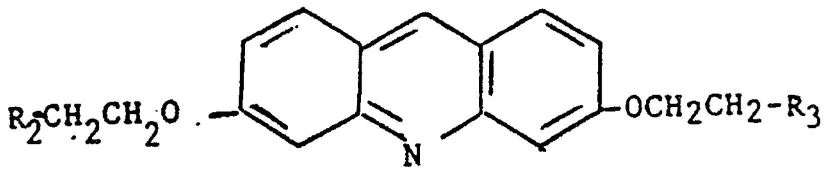

(5)

+

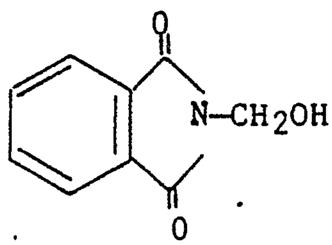

(6)

(7)

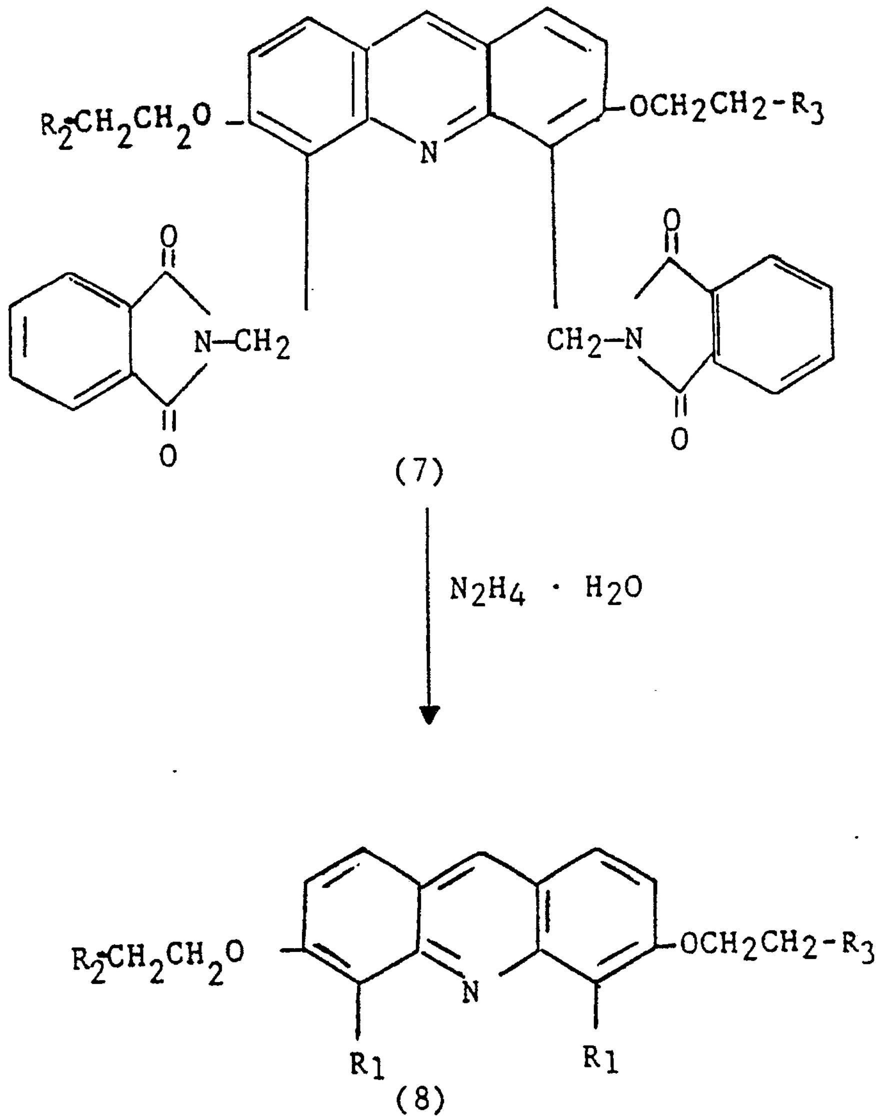


(7)

(8)

In accordance with Flowchart B, a 3,6-bis(substituted)acridine (5), where $R_2$ and $R_3$ are as described above, is dissolved in concentrated sulfuric acid and reacted with hydroxymethyl phthalimide (6) for about 12 to about 24 hours to give (7) which is then reacted with hydrazine hydrate in ethanol at reflux for about 2 to about 4 hours giving (8), where $R_2$ and $R_3$ are as described above and $R_1$ is aminomethyl.

## FLOWCHART C

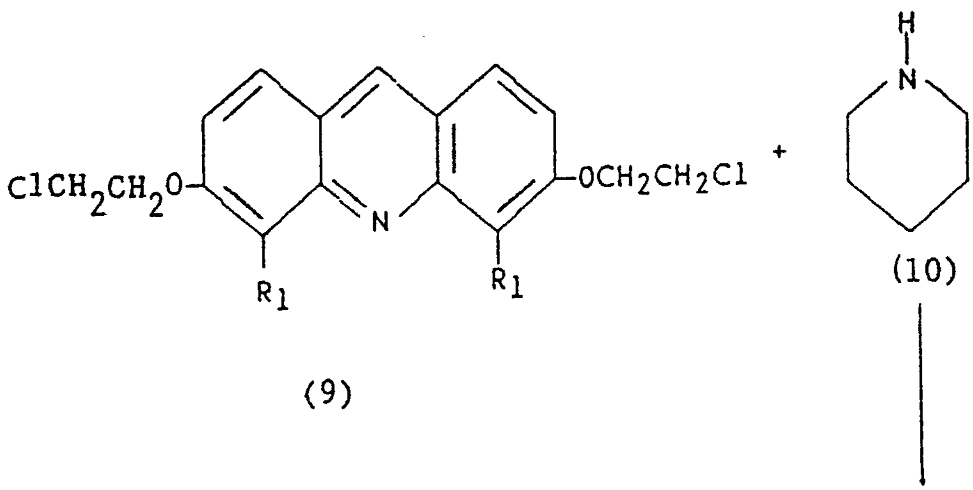

(9)

(10)

(11)

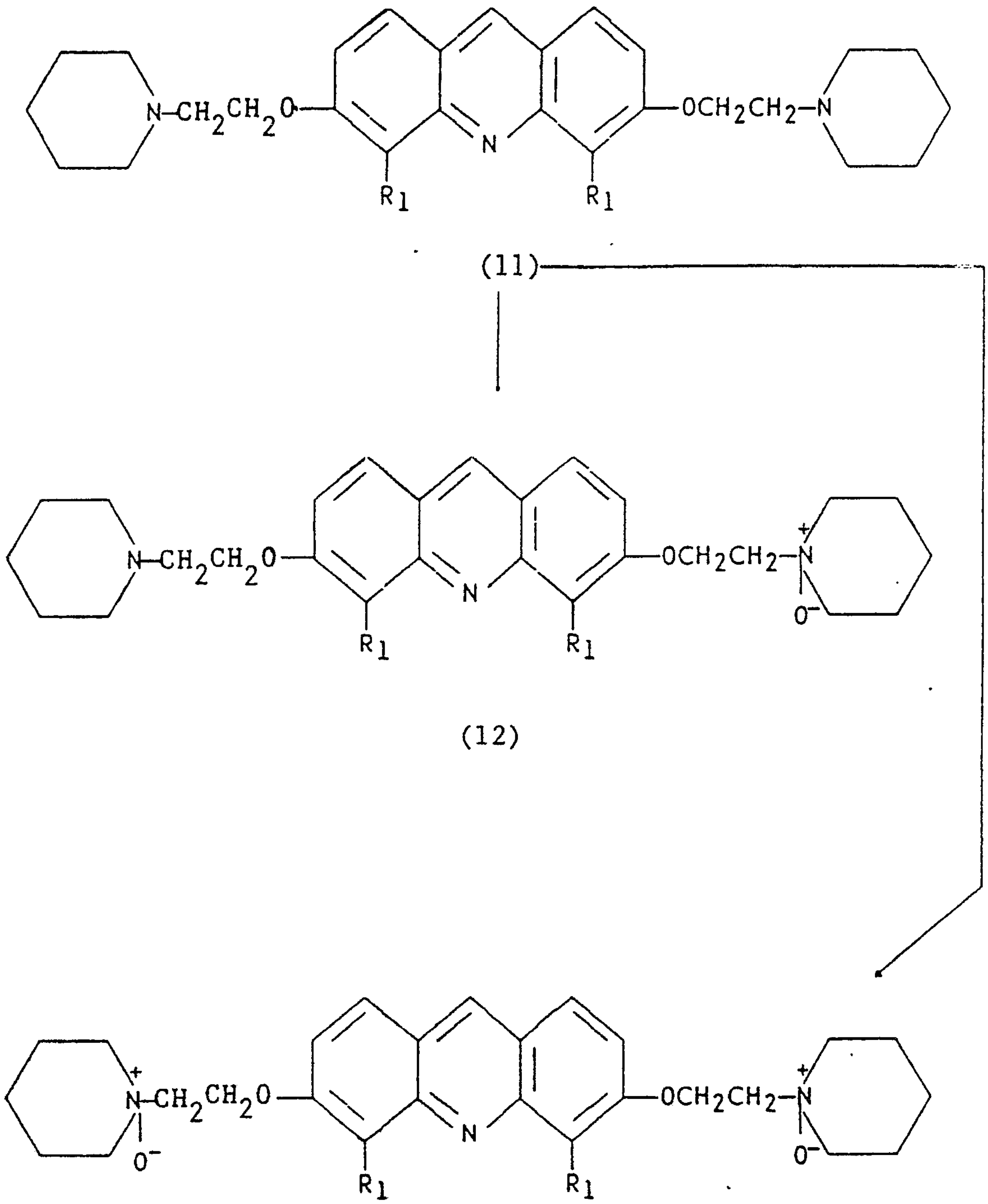

(11)

(12)

(13)

In accordance with the above flowchart, a 3,6-bis(2-chloroethoxy)acridine (9), where $R_1$ is as described hereinabove, is reacted with piperidine in a steel bomb at about 60° to about 100° C for about 18 to about 36 hours, giving a 3,6-bis(2-piperidinoethoxy)-acridine (11) which is then dissolved in methanol, treated with excess 30% hydrogen peroxide for about 12 to about 48 hours, then with platinum catalyst to destroy the excess peroxide and is purified by dry column chromatography on silica gel using the developing system methanol:triethylamine (20:1, v/v). The length of reaction time with hydrogen peroxide is the determining factor in whether a mono-oxide (12) or dioxide (13) is produced.

Further, compounds of the formula:

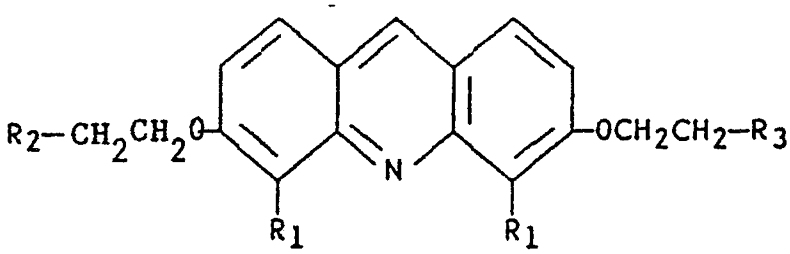

where $R_1$ is as described hereinabove and $R_2$ and $R_3$ are selected from the group consisting of -N-[alkyl-($C_1$-$C_4$)]$_2$ are dissolved in methanol, treated with excess 30% hydrogen peroxide for about 12 to about 48 hours, treated with ether and purified by recrystallization twice from acetonitrile. The length of reaction time with hydrogen peroxide is the determining factor in whether a mono-oxide or dioxide is produced.

The compounds of the present invention have been examined in a variety of murine model systems designed to evaluate their ability tc restore or enhance cellular and humoral immune responses.

In the Rauscher virus model, the ability to produce antibodies to a complex antigen (sheep red blood cells) is severely depressed. The compounds of this invention partially restore functioning of this complex immune system and stimulate antibody production to more normal levels.

The active compounds and novel compositions of the present invention are active as immune modulators when tested according to the following procedures:

(A) Inhibition of Splenomegaly and Restoration of Antibody Formation in Mice with Rauscher Virus-Induced Leukemia

Injection of B ALB/c mice with Rauscher leukemia virus (RLV) is characterized by: 1) a rapidly developing viremia, 2) suppression of the primary antibody response to antigens administered a few days after virus infection, 3) a progressive enlargement of the spleen (splenomegaly), and 4) death resulting from splenic rupture and hemorrhage. The protocol used to infect BALB/c mice with RLV and to test drugs for anticancer and/or immunostimulating activity is as follows:

| | |
|---|---|
| Day 0: | Inject 0.2 ml intraperitoneally (IP) of a 20% (w/v) RLV-infected spleen cell extract into groups of 5 BALB/c mice. The spleen cell extract is prepared from mice infected with RLV 21 days previously. |
| Day +6, +7, +8: | Test compounds are administered orally in 0.5 ml of normal saline containing 0.2% Nobel agar on days +6, +7 and +8. |
| Day +7: | Inject 0.5 ml IP of a thrice saline washed 10% suspension of sheep red blood cells (S-RBC). |
| Day +14: | Bleed mice from the retro-orbital sinus; pool blood from each group. Sacrifice mice, remove and weigh spleens. Serum, harvested from pooled blood of each group of mice, is stored at 40° C for 24 hours. Hemagglutinin tests are performed by standard procedures using a microtiter technique. Acceptable hemagglutinin titer for leukemic (immunosuppressed) mice is ≦1:128. The positive control compound is Poly I:C (polyinosinic acid:polycytidylic acid) administered intraperitoneally on days +6, +7 and +8. Acceptable positive control hemagglutinin titers are 4-fold higher than the titers obtained in leukemic control mice. Average spleen weights of drug treated groups of mice are compared to the average spleen weight of the RLV-infected, placebo treated mice. |

Typical compounds of this invention are active in this test in that they produce a 50% or greater reduction in splenomegaly and a 4-fold or higher increase in hemagglutinin titer to sheep-RBCs relative to the placebo treated, RLV-infected control mice. Results of this test appear in Tables I and II.

## TABLE I

### Rauscher Virus-Induced Leukemia-Percent Reduction in Splenomegaly

| Drug Treatment | Dose (mg/kg) | % Reduction |
|---|---|---|
| 3,6-bis(2-piperidinoethoxy)acridine, N,N-dioxide | 150 | 56 |
| 3,6-bis(2-piperidinoethoxy)acridine, N-oxide | 150 | 76 |
| 3,6-bis(2-diethylaminoethoxy)acridine, N,N-dioxide | 400 | 86 |
| 3,6-bis(2-diethylaminoethoxy)acridine, N,N-dioxide, hydrochloride | 400 | 72 |
| 2,2'-[[3,6-bis[2-(1-piperidinyl)-ethoxy]-4,5-acridinediyl]bis(methylene)]bis-1H-isoindol-1,3(2H)-dione, sulfate (2:5) | 400 | 77 |
| 3,6-bis[2-(diethylamino)ethoxy]-4,5-acridinedimethanol | 400 | 80 |
| 2,2'-[[3,6-bis[2-diethylamino)-ethoxy]-4,5-acridinediyl]bis(methylene)]bis-1H-isoindol-1,3(2H)-dione | 400 | 73 |
| Control, 1,4-bis[(2-aminoethyl)-amino]-5,8-dihydroxyanthraquinone, dihydrochloride (U. S. Patent No. 4,197,249) | 2 | 85 |

TABLE II

Antibody Restoration in Mice with Rauscher Virus-Induced Leukemia

| Drug Treatment | Dose(oral) (mg/kg) | Serum Hemagglutinin Titer / Saline Control Titer (Fold Increase) |
|---|---|---|
| 3,6-bis(2-piperidinoethoxy)-acridine, N,N-dioxide | 150 | 4 |
| 3,6-bis(2-piperidinoethoxy)-acridine, N-oxide | 150 | 16 |
| 3,6-bis(2-diethylaminoethoxy)acridine, N,N-dioxide | 400 | 4 |
| 3,6-bis(2-diethylaminoethoxy)acridine, N,N-dioxide, hydrochloride | 400 | 4 |
| 3,6-bis(2-piperidinoethoxy)--4,5-dichloroacridine, N,N-dioxide | 400 | 4 |
| 3,6-bis(2-piperidinoethoxy)--4,5-dichloroacridine, N,N-dioxide, hydrochloride | 200 | 16 |
| Poly I:C | 10(IP) | 8 |
| 3,6-bis[2-(diethylamino)-ethoxy]-4,5-acridinedimethanol | 400 | 32 |
| 2,2'-[[3,6-bis[2-(diethyl-amino)ethoxy]-4,5-acridinediyl]bis(methylene)]bis-1H-isoindol-1,3(2H)-dione | 400 | 4 |
| Control, Poly I:C | 10 | 4 |

(B) Induction of Circulating Interferon in Mice

Groups of six normal $BDF_1$ male mice were administered a single dose of test compound by the oral, intravenous (IV), intraperitoneal (IP) or subcutaneous (SC) route. Six or eighteen hours later, mice were bled from the retro-orbital sinus and the serum from each group was pooled. Control mice received a 0.2%

Noble agar placebo instead of test compound. Assays of serum interferon were carried out using the semimicroassay of W. E. Stewart (1). The interferon titer of each serum was defined as the reciprocal of the highest dilution of serum that produced a 50% reduction in cytopathic effects of vesicular stomatitis virus (VSV) on monolayers of mouse L-929 cells. The high levels of serum interferon induced in mice, following administration of test compounds, are presented in Table III below.

References to this animal model test system are:

1) The Interferon System. Stewart, W. E., Springer-Verlag, Wien, New York, 1979.

2) Inteferon and Interferon Inducers. Stringfellow, D. A., Marcel Dekker, Inc., New York, 1980.

## TABLE III

### Induction of Circulating Interferon in $BDF_1$ Mice

| Compound | Dose* (mg/kg) | Route | Time After Drug Administration (Hours) | Serum** Interferon Titer |
|---|---|---|---|---|
| 0.3% Klucel® in saline | - | oral | 18 | >100 <320 |
| 3,6-bis(2-Piperidinoethoxy)acridine, N,N-dioxide, hydrochloride | 600 | oral | 18 | 3200 |
| | 300 | oral | 18 | 3200 |
| | 150 | oral | 18 | 3200 |
| | 75 | oral | 18 | 1000 |
| Poly I:C | 10 | IP | 18 | 3200 |
| 0.3% Klucel® in saline | - | oral | 18 | > 100 <320 |
| 3,6-bis(2-Diethylaminoethoxy)acridine, N,N-dioxide | 600 | oral | 18 | 1000 |
| | 300 | oral | 18 | 1000 |
| | 150 | oral | 18 | >1000 <3200 |
| | 75 | oral | 18 | 1000 |
| Poly I:C | 10 | IP | 18 | 3200 |

TABLE III (continued)

| Compound | Dose* (mg/kg) | Route | Time After Drug Administration (Hours) | Serum** Interferon Titer |
|---|---|---|---|---|
| 0.3% Klucel® in saline | - | oral | 18 | >100 <320 |
| 3,6-bis(2-Diethylaminoethoxy)acridine, N,N-dioxide, hydrochloride | 600 | oral | 18 | 3200 |
| | 300 | oral | 18 | 3200 |
| | 150 | oral | 18 | 1000 |
| | 75 | oral | 18 | >320 <1000 |
| Poly I:C | 10 | IP | 18 | 3200 |
| 0.3% Klucel® in saline | - | oral | 18 | >100 <320 |
| 3,6-bis(2-Piperidinoethoxy)-4,5-dichloroacridine, N,N-dioxide | 600 | oral | 18 | 1000 |
| | 300 | oral | 18 | 1000 |
| | 150 | oral | 18 | <10 |
| | 75 | oral | 18 | <10 |
| Poly I:C | 10 | IP | 18 | 3200 |

TABLE III (continued)

| Compound | Dose* (mg/kg) | Route | Time After Drug Administration (Hours) | Serum** Interferon Titer |
|---|---|---|---|---|
| 0.3% Klucel® in saline | - | oral | 18 | >100 <320 |
| 3,6-bis(2-Piperidinoethoxy)-4,5-dichloroacridine, N,N-dioxide, hydrochloride | 600<br>300<br>150<br>75 | oral<br>oral<br>oral<br>oral | 18<br>18<br>18<br>18 | 1000<br>100<br>10<br>< 10 |
| Poly I:C | 10 | IP | 18 | 3200 |
| 0.2% Noble agar | - | oral | 18 | >100 <320 |
| 3,6-bis[2-(diethylamino)ethoxy]-4,5 acridinedimethanol | 400 | oral | 24 | 2100 |
| Control, 3,6-bis(2-piperidinoethoxy)-acridine, trihydrochloride (U.S. Patent No. 4, 314,061) | 400 | oral | 24 | 700 |

* $BDF_1$ male mice received a single dose of test compound, in normal saline, at zero time.

**Reciprocal of serum dilution producing a 50% reduction in cytopathic effects of vesicular stomatitis virus in murine L-929 cells.

(C) Effect of Dose Interval Time on Efficacy of Drugs Against Lethal Virus Challenge of Mice with an Interferon-Sensitive Virus, Columbia SK

Swiss female mice received a single oral dose of test compound or tilorone analog [2,8-bis(N,N-dimethyl-glycyl)dibenzofuran, dihydrochloride (Aldrich Chem. Co.)] on the days indicated in Table IV, prior to lethal subcutaneous virus challenge on day zero with an $LD_{95}$ of Columbia SK virus. The test drugs were suspended in 1.0 ml of 0.2% aqueous agar solution. The test was evaluated 7 days after virus infection. Non-treated controls died with a mean survival time of 4.8 days after infection. The results of this test on typical compounds of this invention appear in Table IV.

The compounds of this invention are authentic modulators of humoral and cellular immunity in mice. The compounds induce high levels of circulating interferon, restore antibody production in immunosuppressed mice, protect against lethal virus infection and stimulate NK-cell cytotoxicity for tumor cells.

It should be understood that this invention relates to modulation of the immune system in warm-blooded animals. Reference herein to animal systems using mice as test subjects is not to be construed as limiting the scope of this invention but rather as illustrative of the efficacy of the compounds of this invention.

It also should be understood that the compounds of this invention used in the above tests and the parameters of the test systems are illustrative.

The method of modulating the immune system of a warm-blooded animal which comprises administering to said animal an effective amount of a medicament, prepared by using a compound of this invention employs methods of treatment, dosage levels and requirements which are well-recognized in the art and may be chosen by those of skill in the art from available methods and techniques.

TABLE IV

Effect of Dose Interval Time on Efficacy of Drugs Against Lethal Challenge of Mice with an Interferon-Sensitive Virus, Columbia SK

| Oral Treatment Time Relative to Virus Challenge (Days) | Number Survivors/Number Treated | | |
|---|---|---|---|
| | 2,2'-[[3,6-bis[2-(1-piperidinyl)ethoxy]-4,5-acridinediyl]bis(methylene)]bis-1H-isoindol-1,3(2H)-dione, sulfate (2:5)(400 mg/kg) | Control Compound (400 mg/kg) | Non-treated Controls |
| 1 day prior | 2/20 | 16/20* | 2/20 |
| | 3,6-bis[2-(diethylamino)ethoxy]acridinedi-methanol (400 mg/kg) | Control Compound (400 mg/kg) | Non-treated Controls |
| 1 day prior | 17/20* | 16/20* | 2/20 |
| | 2,2'-[[3,6-bis[2-(diethylamino)ethoxy]-4,5-acridinediyl]bis(methylene)]bis-1H-isoindol-1,3(2H)-dione (400 mg/kg) | Control Compound (400 mg/kg) | Non-treated Controls |
| 1 day prior | 4/20 | 16/20* | 2/20 |

TABLE IV (continued)

| Oral Treatment Time Relative to Virus Challenge (Days) | Number Survivors/Number Treated | | |
|---|---|---|---|
| | 3,6-bis(2-piperidinoethoxy)-acridine, N,N-dioxide (400 mg/kg) | Tilorone analog (400 mg/kg) | Non-treated Controls |
| -1 | 15/20* | 19/20 | 1/20 |

| Oral Treatment Time Relative to Virus Challenge (Days) | 3,6-bis(2-piperidinoethoxy)-acridine, N-oxide (400 mg/kg) | Tilorone Analog (400 mg/kg) | Non-treated Controls |
|---|---|---|---|
| -1 | 16/20* | 19/20 | 1/20 |

*Indicates significant increase in survival ratio compared to non-treated controls (p <.01).

In order to more fully illustrate the nature of this invention and the manner of practicing same, the following examples are presented.

Example 1

**3,6-Bis(2-piperidinoethoxy)acridine, N-oxide and 3,6-Bis(2-piperidinoethoxy)acridine, N,N-dioxide**

A suspension of 3,6-bis(2-chloroethoxy)acridine hydrochloride in piperidine was heated in a steel bcmb at 80 ° C for 24 hours. The excess piperidine was removed in vacuo and the residual solution was washed twice with 30 ml portions of saturated aqueous sodium bicarbonate, then dried and filtered. The filtrate was evaporated to a residue, giving 3,6-bis(2-piperidinoethoxy)acridine as yellow crystals, mp 129-130 ° C.

A solution of 4.33 g of 3,6-bis(2-piperidinoethoxy)acridine in 50 ml of methanol was treated with 2.3 g of 30% hydrogen peroxide and stirred for 24 hours. At 2 and again at 4 hours, 2.3 g of 30% hydrogen peroxide were added. At the end of 24 hours the reaction was treated with 100 mg of platinum catalyst to destroy the excess hydrogen peroxide. The mixture was filtered and the filtrate concentrated to a yellow gum. This gum was separated into two components by dry column chromatography on a silica gel column developed with methanol:triethylamine (20:1,v/v). The portion of the column containing the Rf 0.06 component was cut out, slurried with methanol, filtered, concentrated to dryness and crystallized from 20 ml of hot water and 100 ml of dioxane, giving 0.53 g of 3,6-bis(2-piperidinoethoxy)acridine, N,N-dioxide as pale yellow crystals, mp 188-190 ° C.

The portion containing the Rf 0.24 component was cut out, slurried with methanol, filtered, concentrated to dryness and crystallized from 20 ml of methanol and 100 ml of water, giving 0.40 g of 3,6-bis(2-piperidinoethoxy)-acridine, N-oxide as colorless crystal, mp 99-100 ° C.

A repeat of the above reaction for 48 rather than 24 hours produced 3.73 g of 3,6-bis(2-piperidinoethoxy)acridine, N,N-oxide which was crystallized from 30 ml of water and 150 ml of dioxane, giving 1.65 g as pale yellow crystals, mp 188-190 ° C.

Example 2

**3,6-Bis(2-piperidinoethoxy)acridine, N,N-dioxide, hydrochloride**

A solution of 5.37 g of 3,6-bis(2-piperidinoethoxy)acridine, N,N-dioxide in 100 ml of 50 ° C water was treated with 3 ml of 5N hydrochloric acid, stirred, treated with 400 ml of dioxane and then cooled rapidly. The resulting precipitate was recovered by filtration, washed with dioxane and dried, giving 5.18 g of the desired product as yellow crystals, mp 155 ° C (dec.).

Example 3

**3,6-Bis(2-diethylaminoethoxy)acridine, N,N -dioxide**

A solution of 12.3 g of 3,6-bis(2-diethylaminoethoxy)acridine free base in 120 ml of methanol was treated with 12 ml of 30% hydrogen peroxide. After 16 hours another 12 ml of 30% hydrogen peroxide was added and then at the end of 24 hours reaction time the solution was treated with 600 ml of ether giving 13 g of crude product. This material was recrystallized twice from acetonitrile, giving 9.05 g of the desired product as a pale yellow solid, mp 94-95 ° C.

Example 4

**3,6-Bis(2-diethylaminoethoxy)acridine, N,N-dioxide, hydrochloride**

A 4.41 g portion of 3,6-bis(2-diethylaminoethoxy)acridine, N,N-dioxide was reacted as described in Example 2, giving 5.02 g of the desired product as yellow crystals, mp 70-72 ° C.

Example 5

**3,6-Bis(2-piperidinoethoxy)-4,5-dichloroacridine, N,N-dioxide**

3,6-Bis(2-piperidinoethoxy)acridine was dissolved in concentrated sulfuric acid. This solution was cooled in an ice bath and N-chlorosuccinimide was added. The mixture was stirred at 0 °C for one hour then at room temperature for 18 hours, poured into ice water and the pH adjusted to 12 with 10N sodium hydroxide. The mixture was extracted with dichloromethane giving a crude material which was crystallized from hexane, giving 3,6-bis(2-piperidinoethoxy)-4,5-dichloroacridine.

A solution of 9.2 g of 3,6-bis(2-piperidinoethoxy)-4,5-dichloroacridine in 2.5 liters of n-propanol at 60 °C, was treated with 75 ml of 30% hydrogen peroxide, with stirring and allowed to cool overnight. Additional 75 ml portions of 30% hydrogen peroxide were added at 24 and 48 hours and the reaction was allowed to proceed for a total of 4 days. The reaction solution was then concentrated to 900 ml and cooled, yielding 7.4 g of crude product. This material was crystallized from 75 ml of water and 400 ml of acetonitrile, giving 2.43 g of the desired product as bright yellow crystals, mp 173-174 °C.

Example 6

**3,6-Bis(2-piperidinoethoxy)-4,5-dichloroacridine, N,N-dioxide, hydrochloride**

A 3.04 g portion of 3,6-bis(2-piperidinoethoxy)-4,5-dichloroacridine, N,N-dioxide was reacted as described in Example 2, giving 3.05 g of the desired product as an orange solid, mp 182-184 °C.

Example 7

**3,6-Bis(2-piperidinoethoxy)-4,5-dimethylacridine, N,N-dioxide**

3,6-Diamino-4,5-dimethylacridine is prepared according to the procedure of Albert, A. and Magrath, D., J. Soc. Chem. Ind., 64, 30 (1945), from 2,6-diaminotoluene, anhydrous formic acid, zinc chloride, glycerol and hydrochloric acid.

3,6-Dihydroxy-4,5-dimethylacridine is prepared by the procedure of Benda, L., Ber., 45, 1787 (1912), for the preparation of 3,6-dihydroxyacridine from 3,6-diaminoacridine. In this reaction 3,6-diamino-4,5-dimethylacridine is heated in a bomb with two parts of water and one part concentrated sulfuric acid at 200 °C for 8 hours. The reaction mixture is diluted with water and the crude solid collected by filtration. This material is purified by dissolution in excess 1N sodium hydroxide, followed by treatment with charcoal and then acidification with acetic acid, giving an orange solid.

A 0.1 mole portion of this 3,6-dihydroxy-4,5-dimethylacridine in 300 ml of dimethylformamide is reacted with 0.11 mole of 50% sodium hydride, with stirring, under nitrogen. After 3 hours, 0.2 mole of N-(2-chloroethyl)-piperidine hydrochloride is added in portions allowing the temperature to rise to 65 °C. The mixture is stirred at room temperature under nitrogen overnight, then filtered and the filtrate concentrated to a residue. This residue is extracted with a mixture of water and methylene chloride. The organic layer is concentrated to an oil which is purified by dissolving in hexane and passing through a short column of alumina. The product is obtained as a yellow crystalline solid, which is 3,6-bis(2-piperidinoethoxy)-4,5-dimethylacridine.

A 4.61 g portion of 3,6-bis(2-piperidinoethoxy)-4,5-dimethylacridine in n-propanol solution is reacted as described in Example 5 with excess hydrogen peroxide, giving the desired product as a yellow solid.

Example 8

**3,6-Bis(2-piperidinoethoxy)-4,5-dimethylacridine, N,N-dioxide, hydrochloride**

A 4.93 g portion of 3,6-bis(2-piperidinoethoxy)-4,5-dimethylacridine, N,N-dioxide is reacted as described in Example 2, giving the desired product as a yellow-orange solid.

Example 9

**3,6-Bis[2-(diethylamino)ethoxy]-4,5-acridine dimethanol, N,N-dioxide, hexahydrate**

To a solution of 0.477 g of 3,6-bis[2-(diethylamino)ethoxyl-4,5-acridine dimethanol in 12 ml of methanol was added 1.0 ml of 30% hydrogen peroxide. The mixture was allowed to stand 24 hours, then 0.5 ml of 30% hydrogen peroxide was added. After standing an additional 16 hours, 50 ml of ether was added giving 0.555 g of the desired product as yellow crystals, mp 166-167 °C.

In order to more fully illustrate the nature of this invention and the manner of practicing same, the following examples are presented.

Example 10

**2,2-[[3,6-Bis[2-(1-piperidinyl)ethoxy]-4,5-acridinediyl]bis(methylene)]bis-1H-isoindol-1,3(2H)-dione, sulfate (2:5)**

A suspension of 3,6-bis(2-chloroethoxy)acridine hydrochloride in piperidine was heated in a steel bomb at 80 °C for 24 hours. The excess piperidine was removed in vacuo and the residual solution was washed twice with 30 ml portions of saturated aqueous sodium bicarbonate and filtered. The filtrate was evaporated to a residue, giving 3,6-bis(2-piperidinoethoxy)acridine as yellow crystals, mp 129-130 °C.

A 7.9 g portion of 3,6-bis(2-piperidinoethoxy)-acridine was dissolved in 50 ml of concentrated sulfuric acid at 40 °C. To this was added 6.5 g of hydroxymethyl phthalimide at 35 °C and the mixture was allowed to stand 17 hours at room temperature. An additional 1.1 g of hydroxymethyl phthalimide was added, the mixture was warmed to 40 °C then allowed to stand for 1.5 hours. The mixture was then poured onto ice, the resulting crystals were collected, dissolved in 200 ml of hot water and then cooled in an ice bath. The crystals were collected and washed with isopropanol, giving 13.8 g of the desired product, mp 190-205 °C.

Example 11

**3,6-Bis[2-(diethylamino)ethoxy]-4,5-acridinedimethanol**

To a solution of 23.43 g of 3,6-bis(2-diethylaminoethoxy)acridine trihydrochloride (U. S. Patent No. 3,740,403) in 85 ml of concentrated sulfuric acid was added 2.94 g of paraformaldehyde. The mixture was stirred 45 minutes, then added to 1.5 liters of ether. The resulting solid was collected, dissolved in water, sodium bicarbonate was added and the mixture extracted with six 75 ml portions of chloroform. These extracts were placed on an alumina column which was then eluted with ether and then methylene chloride. The methylene chloride extract was concentrated, giving 4.0 g of the desired product as yellow crystals, mp 95-96 °C.

Example 12

**2,2'-[[3,6-Bis[2-(diethylamino)ethoxy]-4,5-acridinediyl]bis(methylene)]bis-1H-isoindol-1,3(2H)-dione**

To a solution of 15.72 g of 3,6-bis(2-diethylaminoethoxy)acridine trihydrochloride in 70 ml of concentrated sulfuric acid was added 11.0 g of hydroxymethyl phthalimide. The mixture was allowed to stand 18 hours and then poured onto ice. The resulting solid was collected, dissolved in hot chloroform, dried and concentrated. The resulting yellow crystals were collected and washed with ether, giving 14 g of the desired product, mp 242-244 °C.

Example 13

**3,6-Bis[2-(diethylamino)ethoxy]-4,5-acridinedimethanamine, tetrahydrochloride**

A solution of 1.52 g of 2,2'-[[3,6-bis[2-(diethylamino)ethoxy]-4,5-acridinediyl]bis(methylene)]bis-1H-isoindol-1,3(2H)-dione, 40 ml of ethanol and 1.4 ml of hydrazine hydrate was refluxed for 3 hours, cooled and filtered. The filtrate was concentrated, refiltered and to this filtrate was added sodium hydroxide. The resulting gummy orange solid was dissolved in chloroform, concentrated, isopropanol and hydrochloric acid were added and the resulting solid collected, dissolved in hot methanol and cooled, giving 593 mg of the desired product, mp 271-280 °C (dec.).

Example 14

**3,6-Bis[2-(diethylamino)ethoxy]-4,5-diformylacridine**

To a solution of 0.469 g of 3,6-bis[2-(diethylamino)ethoxy]-4,5-acridinedimethanol in a mixture of 5.0 ml of anhydrous dimethyl sulfoxide and 2.8 ml of triethylamine is added a solution of 0.96 g of sulfur trioxide-pyridine complex in 5 ml of anhydrous dimethyl sulfoxide. The reaction mixture is stirred at room temperature for one hour. Most of the dimethyl sulfoxide is removed under reduced pressure without heat. The residue is treated first with 50 ml of saturated sodium bicarbonate solution and then with 50 ml of water, giving 0.35 g of the desired product as a yellow solid.

## Claims

**1.** A compound selected from the group consisting of those of the formula

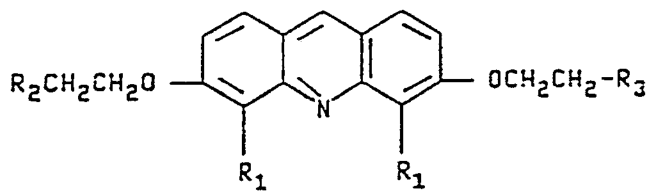

wherein $R_1$ is formyl, hydroxymethyl, aminomethyl, the group:

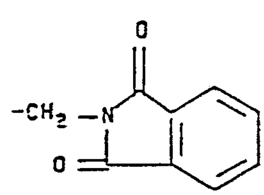

,

hydrogen, halogen, -COOH, and alkyl ($C_1$-$C_3$), $R_2$ and $R_3$ may be the same or different and are piperidinyl, -N[alkyl ($C_1$-$c_5$)]$_2$, pyrrolidino, morpholino, or the groups:

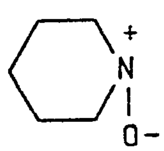

or

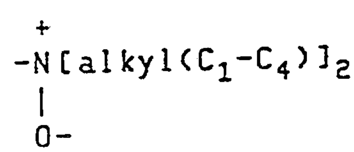

with the provisos that where $R_1$ is aminomethyl or

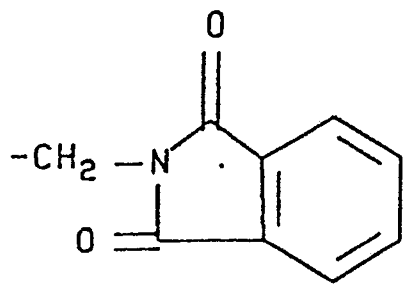

,

$R_2$ and $R_3$ must be pyrrolidino, morpholino, piperidinyl, or N-[alkyl($C_1$-$C_5$)]$_2$;and where $R_1$ is halogen, -COOH or alkyl($C_1$-$C_3$) then $R_2$ and $R_3$ must be piperidinyl or

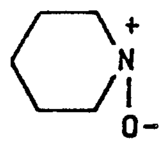 ,

and where R1 is hydrogen, R2 and R3 may be the same or different and are piperidinyl, piperidinyl-N-oxide, $-N(C_2H_5)_2$ or

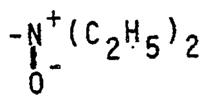

with the proviso that at least one of $R_2$ and $R_3$ must be in the N-oxide form; and the pharmaceutically acceptable salts thereof.

2. A compound according to Claim 1 wherein $R_1$ is selected from the group consisting of hydrogen and hydroxymethyl, and $R_2$ and $R_3$ may be the same or different and are

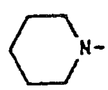 , 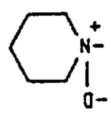 ,

[$-N(C_2H_5)_2$ or 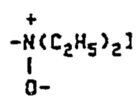

with the proviso that at least one of $R_2$ and $R_3$ must be in the N-oxide form.

3. A compound according to Claim 1 wherein $R_2$ and $R_3$ are piperidinyl; $R_1$ is formyl, hydroxymethyl,aminomethyl, or

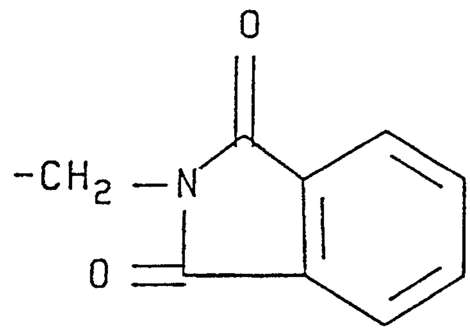

4. A compound according to Claim 1, wherein the compound is 2,2'-[[3,6-bis[2-(1-piperidinyl)ethoxy]-4,5-acridinediyl]bis-(methylene)]bis-1H-isoindol-1,3(2H)-dione; 3,6-bis[2-(diethyl-amino)ethoxy]-4,5-acridine-dimethanol; 2,2'-[[3,6-bis[2-(diethylamino)ethoxy]-4,5-acridinediyl]bis(methylene)]bis-1H-isoindol-1,3-(2H)-dione; 3,6-bis[2-(diethylamino)ethoxy]-4,5-acridine dimethanamine, tetrahydrochloride; or 3,6-bis-[2-(diethylamino)ethoxy]-4,5-diformylacridine.

5. A compound according to claim 1, wherein the compound is 3,6-bis(2-piperidinoethoxy)acridine, N,N-dioxide; 3,6-bis(2-piperidinoethoxy)acridine, N,N-dioxide; hydrochloride; 3,6-bis(2-diethylaminoethoxy)-acridine, N,N-dioxide or 3,6-bis(2-diethylaminoethoxy)acridine, N,N-dioxide, hydrochloride.

6. A compound according to claim 1, wherein the compound is 3,6-bis(2-piperidinoethoxy)-4,5-dichloroacridine, N,N-dioxide; 3,6-bis(2-piperidinoethoxy)-4,5-dichloroacridine, N,N-dioxide, hydrochloride; 3,6-bis(2-piperidinoethoxy)-4,5-dimethylacridine, N,N-dioxide; 3,6-bis(2-piperidinoethoxy)-4,5-dimethylacridine, N,N-dioxide, hydrochloride or 3,6-bis[2(diethylamino)ethoxy]-4,5-acridine dimethanol, N,N-dioxide.

7. A process for the preparation of a compound of the following formula:

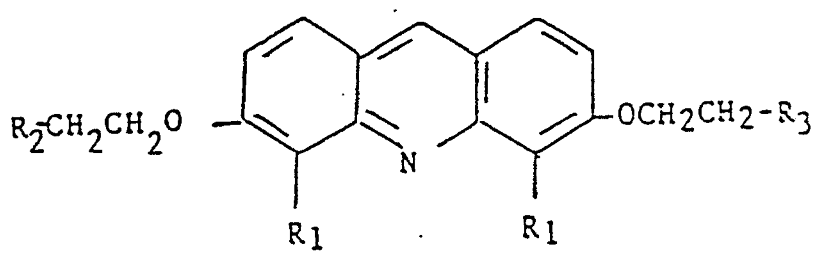

wherein $R_1$ is formyl and hydroxymethyl, $R_2$ and $R_3$ are selected from the group consisting of piperidinyl, -N(alkyl($C_1$-$C_5$))$_2$, pyrrolidino and morpholino, characterized by reacting a 3,6-bis-(substituted)acridine of the formula:

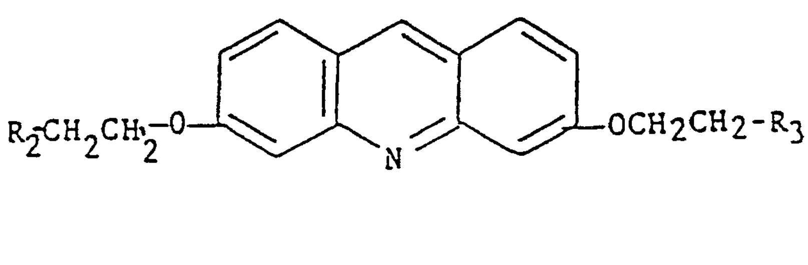

,

wherein $R_2$ and $R_3$ are as defined above, in concentrated sulfuric acid with paraformaldehyde; adding ether to yield compounds of the formula:

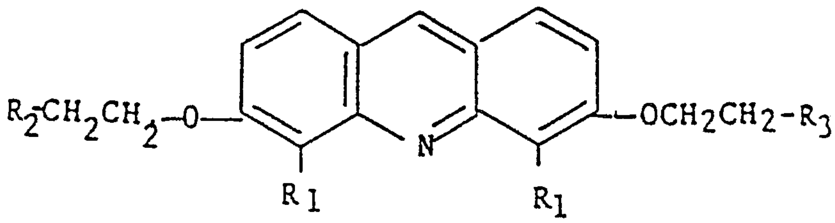

wherein $R_2$ and $R_3$ are as described above and $R_1$ is $-CH_2OH$; dissolving said compounds in dimethylsulfoxide; adding triethylamine; and reacting with sulfur trioxide-pyridine complex to yield the compounds of the above formula wherein $R_2$ and $R_3$ are as defined above and $R_1$ is formyl.

**8.** A process for producing a compound of the formula:

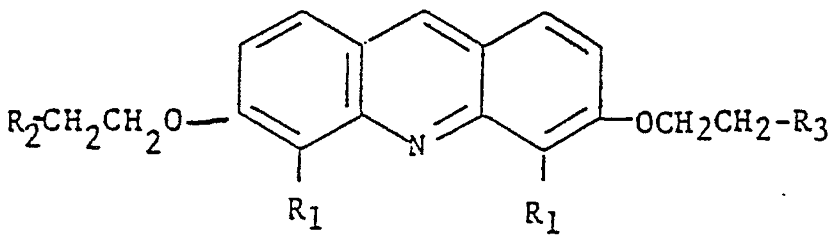

wherein $R_2$ and $R_3$ are piperidinyl, $-N[alkyl(C_1-C_5)]_2$, pyrrolidine or morpholino; and $R_1$ is aminomethyl or

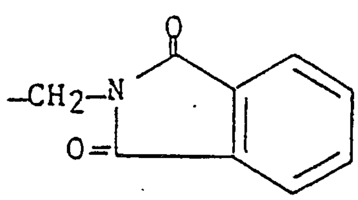

characterized by reacting a 3,6-bis(substituted)acridine of the formula:

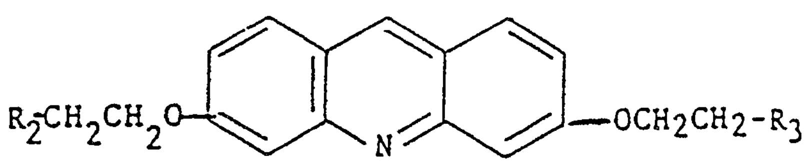

wherein $R_2$ and $R_3$ are as defined above, in concentrated sulfuric acid with hydroxymethyl phthalimide, to yield compounds of the formula:

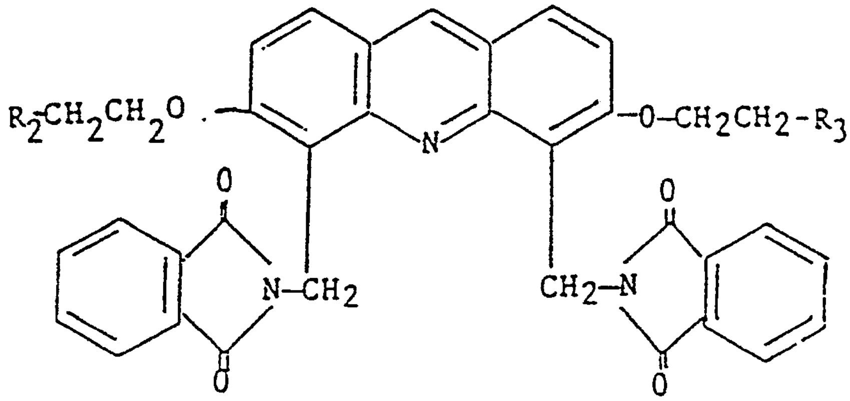

; and

reacting said compounds with hydrazine hydrate in ethanol at reflux for about 2 to about 4 hours to yield compounds of the formula:

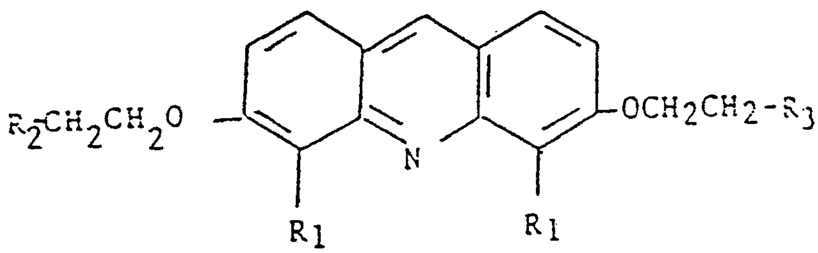

wherein $R_2$ and $R_3$ are as defined above and $R_1$ is aminomethyl.

9. A process for preparing a compound of the formula:

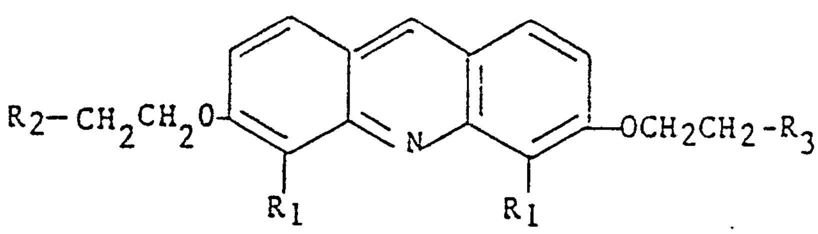

wherein $R_1$ is selected from the group consisting of hydrogen, halogen, hydroxymethyl, formyl, -COOH and alkyl ($C_1$-$C_3$); $R_2$ and $R_3$ may be the same or different and are selected from the group consisting of

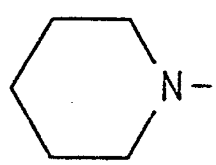

and

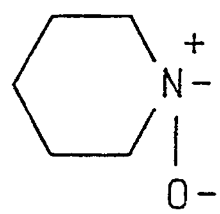

with the proviso that one of $R_2$ and $R_3$ must be in the N-oxide form, characterized by reacting a compound of the formula

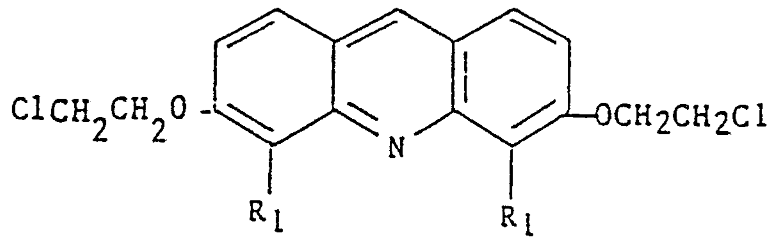

where $R_1$ is as defined above with piperidine in a steel bomb at about 60° C to about 100° C to yield a 3,6-bis(2-piperidinoethoxy)acridine of the formula:

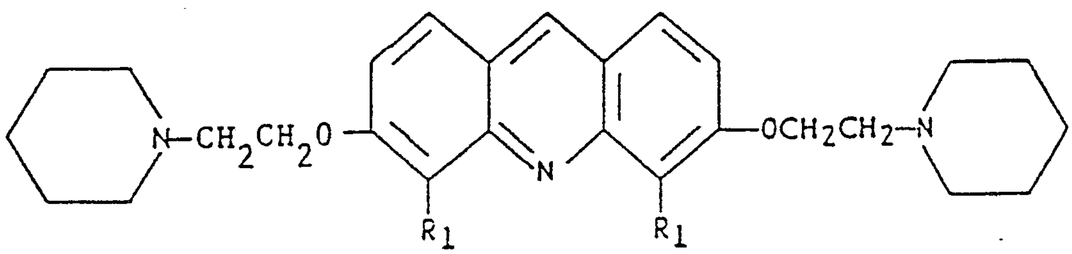

dissolving said compounds in methanol; treating with excess 30% hydrogen peroxide; treating with platinum catalyst; and purifying the compounds by dry column chromatography to yield, depending upon the reaction time, the mono-N-oxide or N,N-dioxide derivative.

**10.** A process for preparing a compound as defined in claim 1 of the formula:

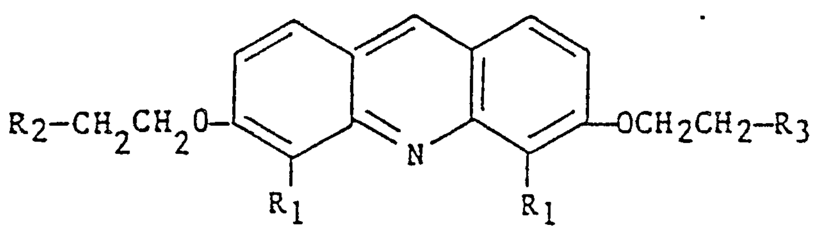

wherein $R_1$ is selected from the group consisting of hydrogen, halogen, hydroxymethyl, formyl, -COOH and alkyl ($C_1$-$C_3$); $R_2$ and $R_3$ may be the same or different and are selected from the group consisting of -N-[alkyl($C_1$-$C_4$)]$_2$and

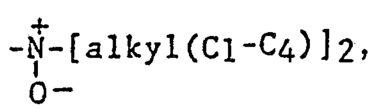

with the proviso that at least one of $R_2$ and $R_3$ must be in the N-oxide form, characterized by dissolving a compound of the formula:

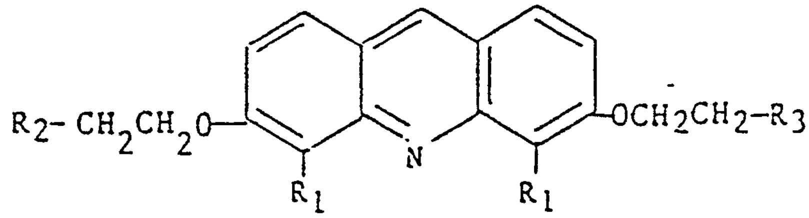

where $R_1$ is as defined above and $R_2$ and $R_3$ are -N-[alkyl-($C_1$-$C_4$)]$_2$ in methanol; treating with excess 30% hydrogen peroxide; treating with ether; and purifying by recrystallization from acetonitrile to yield, depending upon reaction time, the mono-N-oxide or N,N-dioxide derivative.

11. A process for preparing a compound of the formula:

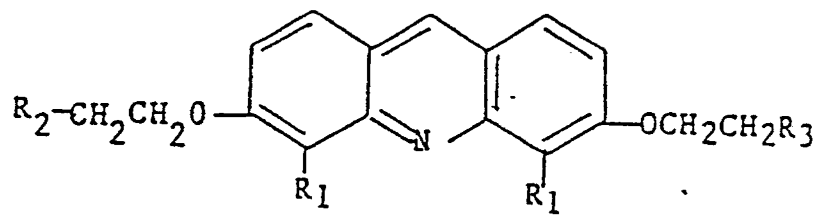

wherein $R_1$ is of chloro and methyl; $R_2$ and $R_3$ may be the same or different and are

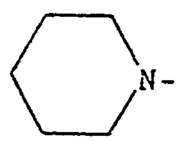

or

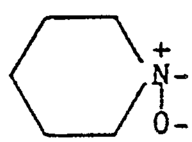

,

with the proviso that at least one of $R_2$ and $R_3$ must be in the N-oxide form; characterized by dissolving a compound of the formula:

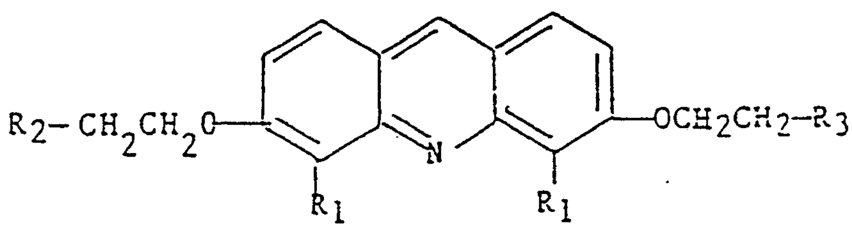

where $R_1$ is as defined above and $R_2$ and $R_3$ are

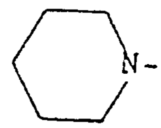

in n-propanol; treating with excess 30% hydrogen peroxide and crystallizing from water and acetonitrile to yield, depending upon reaction time, the mono-N-dioxide or N,N-dioxide derivative.

## Revendications

**1.** Composé, choisi dans l'ensemble consistant en les composés de formule :

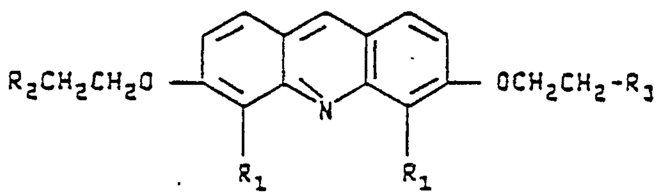

dans laquelle $R_1$ représente un groupe formyle, hydroxyméthyle, aminométhyle, le groupe :

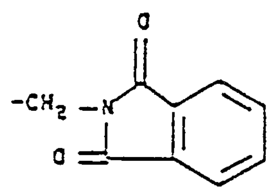

,

un atome d'hydrogène ou d'halogène, un groupe -COOH et alkyle (en $C_1$ à $C_3$) ; $R_2$ et $R_3$ peuvent être identiques ou différents et ils représentent chacun un groupe pipéridinyle, -N[alkyle (en $C_1$ à $C_5$)]$_2$, pyrrolidino, morpholino, ou les groupes :

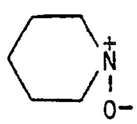

ou

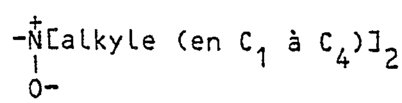

à la condition que, lorsque $R_1$ représente un groupe aminométhyle ou

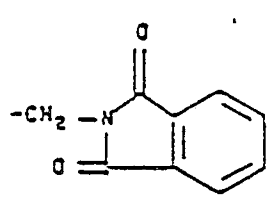

,

$R_2$ et $R_3$ doivent représenter chacun un groupe pyrrolidino, morpholino, pipéridinyle ou N-[alkyle (en $C_1$ à $C_5$)]$_2$ ; et lorsque $R_1$ représente un atome d'halogène, un groupe -COOH ou alkyle (en $C_1$ à $C_3$), $R_2$ et $R_3$ doivent représenter chacun un groupe pipéridinyle ou

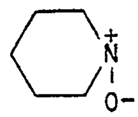 ,

et lorsque $R_1$ représente un atome d'hydrogène, $R_2$ et $R_3$ peuvent être identiques ou différents et ils représentent chacun un groupe pipéridinyle, N-oxyde de pipéridinyle, $-N(C_2H_5)_2$ ou

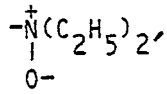

à la condition qu'au moins l'un des groupes $R_2$ et $R_3$ soit sous forme N-oxyde ; et ses sels pharmaceutiquement acceptables.

**2.** Composé selon la revendication 1, dans lequel $R_1$ est choisi dans l'ensemble constituant d'une part un atome d'hydrogène et un groupe hydroxyméthyle ; et $R_2$ et $R_3$ peuvent être identiques ou différents et ils représentent chacun

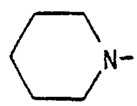 , 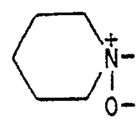 ,

[$-N(C_2H_5)_2$ ou 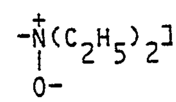 .

à la condition qu'au moins l'un des groupes $R_2$ et $R_3$ soit sous forme de N-oxyde.

**3.** Composé selon la revendication 1, dans lequel $R_2$ et $R_3$ représentent chacun un groupe pipéridinyle ; $R_1$ représente un groupe formyle, hydroxyméthyle, aminométhyle, ou

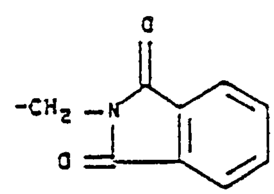 ,

**4.** Composé selon la revendication 1, ce composé étant la 2,2'-[[3,6-bis[2-(1-pipéridinyl)éthoxy]-4,5-acridinediyl]bis-(méthylène)]bis-1H-isoindole-1,3(2H)-dione ; le 3,6-bis[2-(diéthylamino)éthoxy]-4,5-acridine-diméthanol ; la 2,2'-[[3,6-bis[2-diéthylamino)éthoxy]-4,5-acridinediyl]bis(méthylène)]bis-1H-isoindole-1,3(2H)-dione ; le tétrachlorhydrate de 3,6-bis[2-(diéthylamino)éthoxy]-4,5-acridine diméthanamine ; ou la 3,6-bis-[2-(diéthylamino)éthoxy]-4,5-diformylacridine.

**5.** Composé selon la revendication 1, ce composé étant le N,N-dioxyde de la 3,6-bis(2-pipéridinoéthoxy)-acridine ; le chlorhydrate de N,N-dioxyde de 3,6-bis(2-pipéridinoéthoxy)acridine ; le N,N-dioxyde de la 3,6-bis(2-diéthylaminoéthoxy)acridine ou le chlorhydrate du N,N-dioxyde de la 3,6-bis(2-diéthylaminoéthoxy)-acridine.

6. Composé selon la revendication 1, ce composé étant le N,N-dioxyde de la 3,6-bis(2-pipéridinoéthoxy)-4,5-dichloroacridine ; le chlorhydrate du N,N-dioxyde de la 3,6-bis(2-pipéridinoéthoxy)-4,5-dichloroacridine ; le N,N-dioxyde de la 3,6-bis(2-pipéridinoéthoxy)-4,5-diméthylacridine ; le chlorhydrate du N,N-dioxyde de la 3,6-bis(2-pipéridinoéthoxy)-4,5-diméthylacridine ou le N,N-dioxyde du 3,6-bis[2-(diéthylamino)éthoxy]-4,5-acridine diméthanol.

7. Procédé pour préparer un composé répondant à la formule suivante :

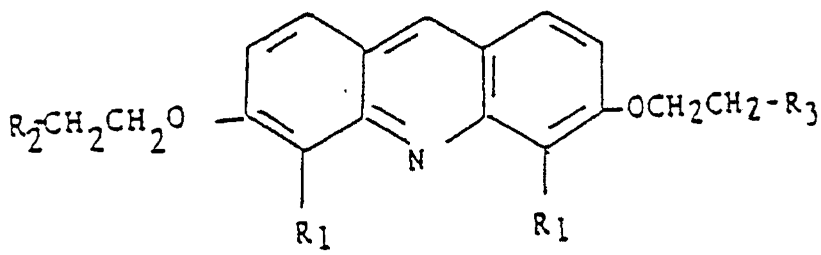

dans laquelle $R_1$ représente un groupe formyle et hydroxyméthyle ; $R_2$ et $R_3$ sont choisis dans l'ensemble consistant en un groupe pipéridinyle, -N[alkyle (en $C_1$ à $C_5$)]$_2$, pyrrolidino et morpholino, procédé caractérisé en ce qu'on fait réagir une acridine bis(substituée) en 3,6, de formule :

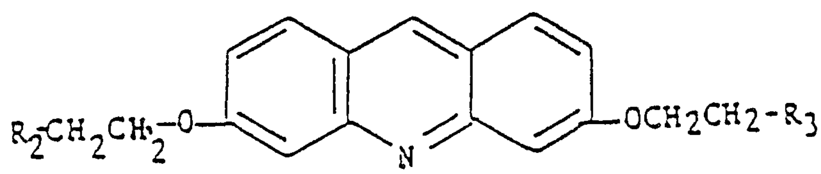

dans laquelle $R_2$ et $R_3$ répondent à la définition ci-dessus, dans de l'acide sulfurique concentré avec du paraformaldéhyde ; on ajoute de l'éther, ce qui donne des composés de formule :

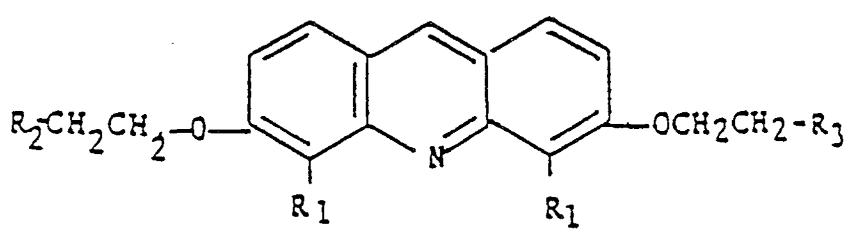

dans laquelle $R_2$ et $R_3$ sont tels que décrits ci-dessus ; et $R_1$ représente $-CH_2OH$ ; on dissout lesdits composés dans du diméthylsulfoxyde ; on ajoute de la triéthylamine ; et l'on fait réagir avec un complexe trioxyde de soufre/pyridine, ce qui donne les composés répondant à la formule ci-dessus dans laquelle $R_2$ et $R_3$ sont tels que définis ci-dessus et $R_1$ représente un groupe formyle.

8. Procédé pour produire un composé de formule :

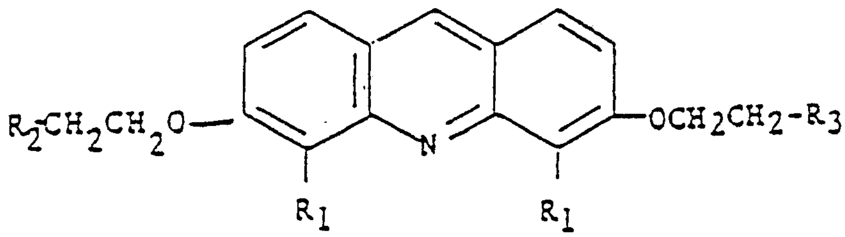

dans laquelle $R_2$ et $R_3$ représentent chacun un groupe pipéridinyle, -N[alkyle (en $C_1$ à $C_5$]$_2$, pyrrolidino ou morpholino ; et $R_1$ représente un groupe aminométhyle ou

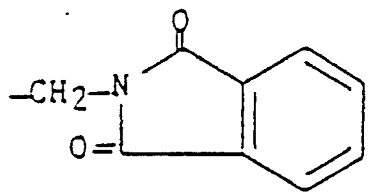

procédé caractérisé en ce qu'on fait réagir une acridine bis(substituée) en 3,6, de formule

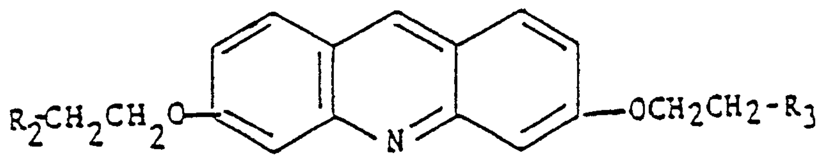

dans laquelle $R_2$ et $R_3$ sont tels que définis ci-dessus, dans de l'acide sulfurique concentré, avec de l'hydroxyméthyphtalimide, ce qui donne des composés de formule :

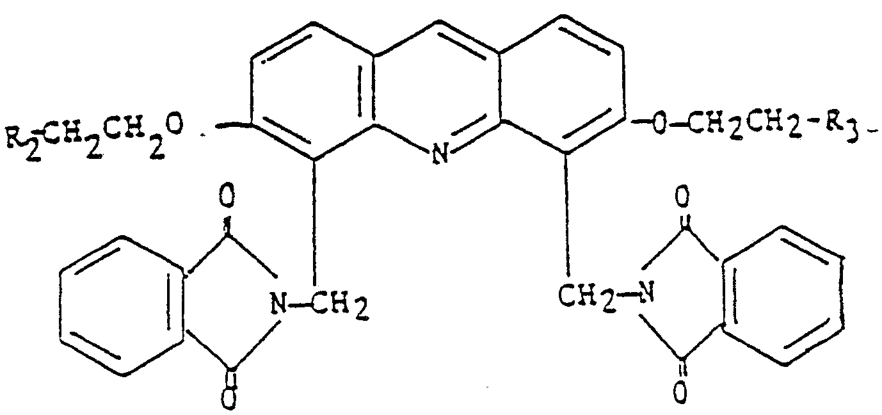

et l'on fait réagir lesdits composés avec de l'hydrate d'hydrazine dans de l'éthanol, au reflux durant environ 2 à environ 4 h, ce qui donne des composés de formule :

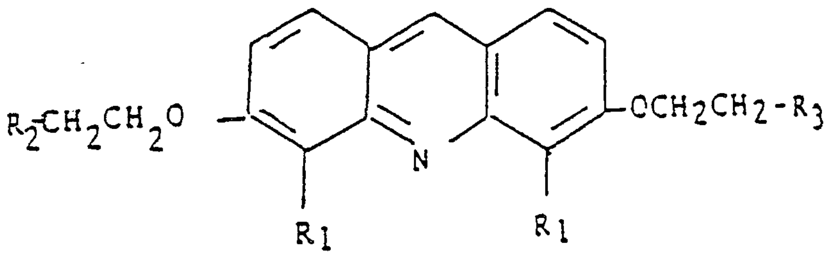

dans laquelle $R_2$ et $R_3$ sont tels que définis ci-dessus et $R_1$ représente un groupe aminométhyle.

9. Procédé pour préparer un composé de formule :

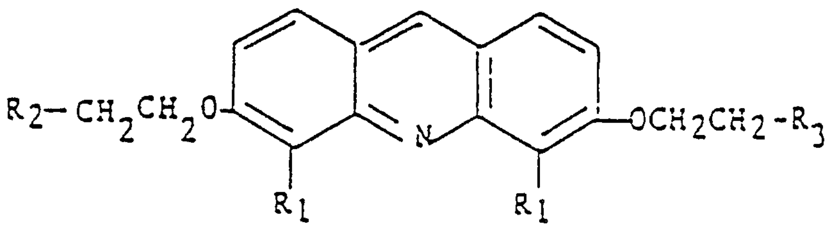

dans laquelle $R_1$ est choisi dans l'ensemble constitué par un atome d'hydrogène ou d'halogène, un groupe hydroxyméthyle, formyle, -COOH et alkyle (en $C_1$ à $C_3$) ; $R_2$ et $R_3$ peuvent être identiques ou différents et ils sont choisis chacun dans l'ensemble consistant en

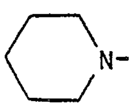

et

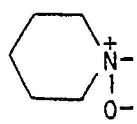

à la condition que l'un des symboles $R_2$ et $R_3$ soit sous forme de N-oxyde, procédé caractérisé en ce qu'on fait réagir un composé de formule :

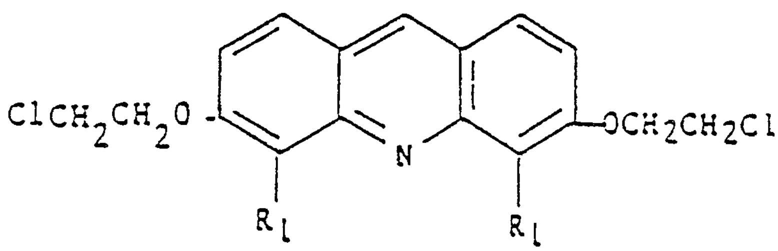

dans laquelle $R_1$ est tel que défini ci-dessus avec de la pipéridine dans une bombe en acier, à environ 60 °C jusqu'à environ 100 °C, ce qui donne une 3,6-bis(2-pipéridinoéthoxy)acridine de formule :

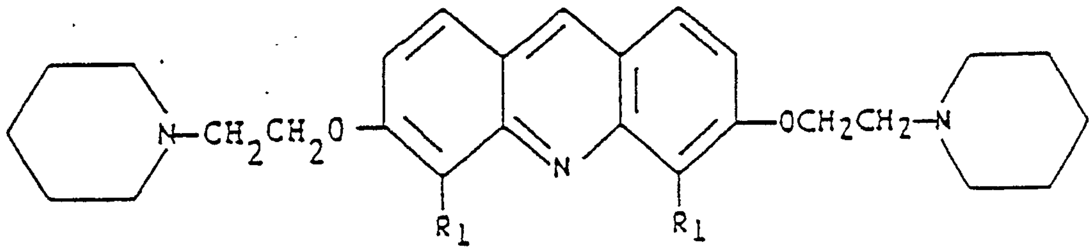

on dissout lesdits composés dans du méthanol ; on traite par un excès de peroxyde d'hydrogène à 30 % ; on traite par un catalyseur au platine ; et l'on purifie les composés par chromatographie sur colonne sèche, ce qui donne, selon le temps de réaction, le mono-N-oxyde ou le N,N-dioxyde dérivé.

**10.** Procédé pour préparer un composé tel que défini à la revendication 1, de formule :

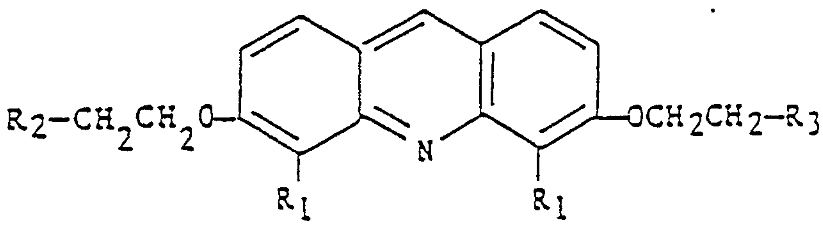

dans laquelle $R_1$ est choisi dans l'ensemble consistant en un atome d'hydrogène ou d'halogène, un groupe hydroxyméthyle, formyle, -COOH et alkyle (en $C_1$ à $C_3$) ; $R_2$ et $R_3$ peuvent être identiques ou différents et ils sont choisis chacun dans l'ensemble consistant en un groupe -N-[alkyle (en $C_1$ à $C_4$)]$_2$ et

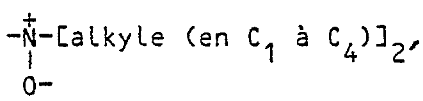

à la condition qu'au moins l'un des groupes $R_2$ et $R_3$ soit sous forme de N-oxyde, procédé caractérisé en ce qu'on dissout un composé de formule :

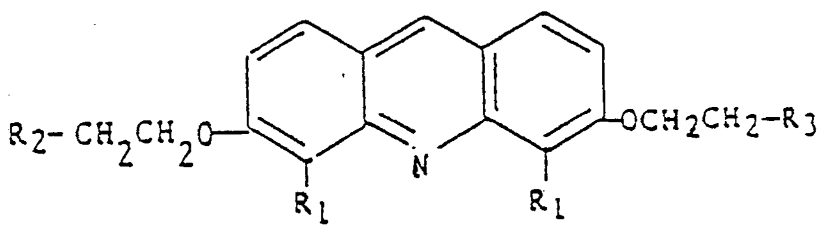

dans laquelle $R_1$ est tel que défini ci-dessus, et $R_2$ et $R_3$ représentent chacun un groupe -N-[alkyle (en $C_1$ à $C_4$)]$_2$ dans du méthanol ; on traite par un excès de peroxyde à 30 % ; et on traite par de l'éther ; et l'on purifie par recristallisation à partir d'acétonitrile, ce qui donne, selon le temps de réaction, le mono-N-oxyde ou le N,N-dioxyde dérivé.

**11.** Procédé pour préparer un composé de formule :

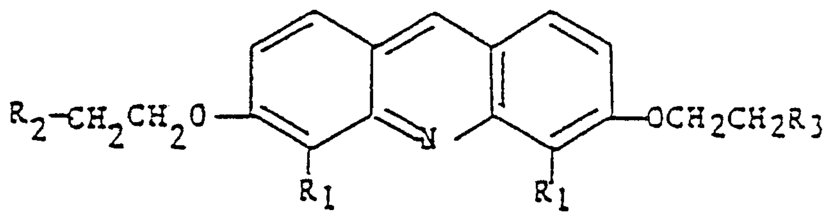

dans laquelle $R_1$ représente un groupe chloro ou méthyle ; $R_2$ et $R_3$ peuvent être identiques ou différents et représentent chacun

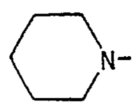

ou

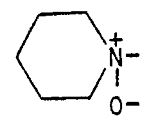

,

à la condition qu'au moins l'un des $R_2$ et $R_3$ soit sous forme de N-oxyde, procédé caractérisé en ce qu'on dissout un composé de formule :

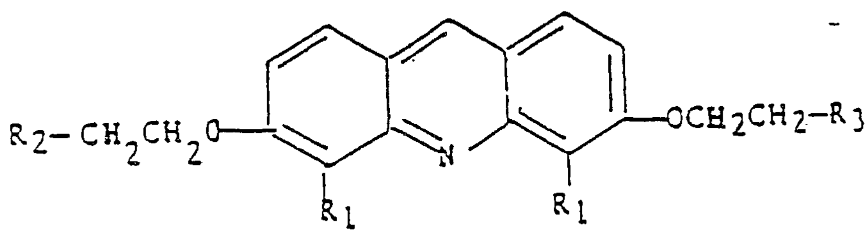

dans laquelle $R_1$ est tel que défini ci-dessus, et $R_2$ et $R_3$ représentent chacun

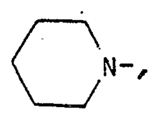

dans du n-propanol ; on traite par un excès de peroxyde d'hydrogène à 30 % et l'on fait cristalliser à partir d'eau et d'acétonitrile, ce qui donne, selon le temps de réaction, le mono-N-dioxyde ou le N,N-dioxyde dérivé.

## Ansprüche

1. Verbindung, ausgewählt aus der Gruppe bestehend aus solchen der Formel

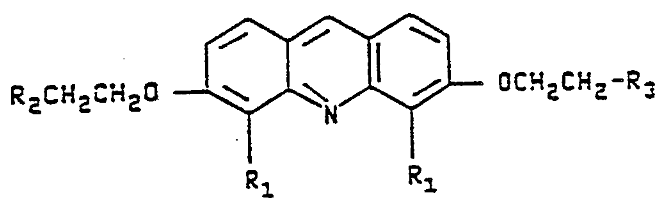

wobei $R_1$ für Formyl, Hydroxymethyl, Aminomethyl, die Gruppe

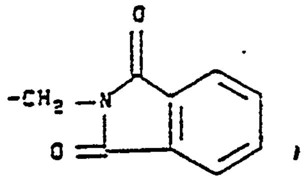

Wasserstoff, Halogen, -COOH und Alkyl ($C_1$-$C_3$) steht, $R_2$ und $R_3$ gleich oder verschieden sein können und für Piperidinyl, -N[Alkyl($C_1$-$C_5$)]$_2$, Pyrrolidino, Morpholino oder die Gruppen

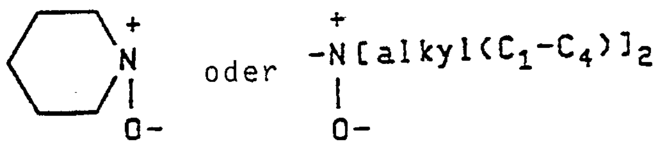

stehen, mit den Maßgaben, daß dann, wenn $R_1$ für Aminomethyl oder

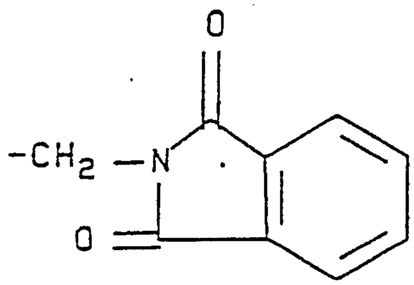

steht, $R_2$ und $R_3$ Pyrrolidino, Morpholino, Piperidinyl oder N-[Alkyl($C_1$-$C_5$)]$_2$ sein müssen, und daß dann, wenn $R_1$ für Halogen, -COOH oder Alkyl($C_1$-$C_3$) steht, $R_2$ und $R_3$ Piperidinyl oder

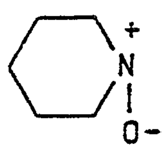

sein müssen und daß dann, wenn $R_1$ für Wasserstoff steht, $R_2$ und $R_3$ gleich oder verschieden sein können und Piperidinyl, Piperidinyl-N-oxid, -N($C_2H_5$)$_2$ oder

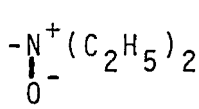

bedeuten, mit der Maßgabe, daß mindestens eines von $R_2$ und $R_3$ in der N-Oxidform vorliegen muß; und die pharmazeutisch akzeptablen Salze derselben.

2. Verbindung gemäß Anspruch 1, wobei $R_1$ ausgewählt ist aus der Gruppe bestehend aus Wasserstoff und Hydroxymethyl und $R_2$ und $R_3$ gleich oder verschieden sein können und für

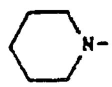 , 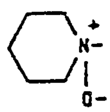 ,

$-N(C_2H_5)_2$ oder 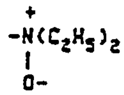

stehen, mit der Maßgabe, daß mindestens eines von $R_2$ und $R_3$ in der N-Oxidform vorliegen muß.

3. Verbindung gemäß Anspruch 1, wobei $R_2$ und $R_3$ Piperidinyl sind, $R_1$ für Formyl, Hydroxymethyl, Aminomethyl oder

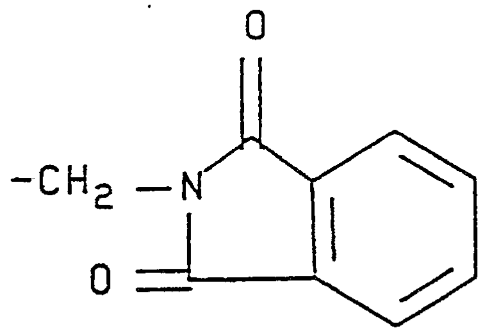

steht.

4. Verbindung gemäß Anspruch 1, nämlich 2,2'-[[3,6-Bis-[2-(1-piperidinyl)ethoxy]-4,5-acridindiyl]bis-(methylen)]bis-1H-isoindol-1,3(2H)-dion; 3,6-Bis-[2-(diethylamino)ethoxy]-4,5-acridindimethanol; 2,2'-[-[3,6-Bis-[2-(diethylamino)ethoxy]-4,5-acridindiyl]bis(methylen)]bis-1H-isoindol-1,3(2H)-dion; 3,6-Bis-[2-(diethylamino)ethoxy]-4,5-acridindimethanamin, Tetrahydrochlorid; oder 3,6-Bis-[2-(diethylamino)-ethoxy]-4,5-diformylacridin.

5. Verbindung gemäß Anspruch 1, nämlich 3,6-Bis-(2-piperidinoethoxy)acridin, N,N-Dioxid; 3,6-Bis-(2-piperidinoethoxy)-acridin, N,N-Dioxid, Hydrochlorid; 3,6-Bis-(2-diethylaminoethoxy)acridin, N,N-Dioxid oder 3,6-Bis-(2-diethylaminoethoxy)-acridin, N,N-Dioxid, Hydrochlorid.

6. Verbindung gemäß Anspruch 1, nämlich 3,6-Bis-(2-piperidinoethoxy)-4,5-dichloracridin, N,N-Dioxid; 3,6-Bis-(2-piperidinoethoxy)-4,5-dichloracridin, N,N-Dioxid, Hydrochlorid; 3,6-Bis-(2-piperidinoethoxy)-4,5-dimethylacridin, N,N-Dioxid; 3,6-Bis-(2-piperidinoethoxy)-4,5-dimethylacridin, N,N-Dioxid, Hydrochlorid oder 3,6-Bis-[2-(diethylamino)ethoxy]-4,5-acridindimethanol, N,N-Dioxid.

7. Verfahren zur Herstellung einer Verbindung der folgenden Formel

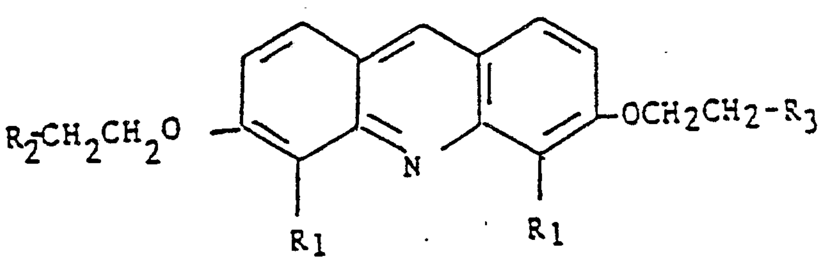

wobei $R_1$ für Formyl und Hydroxymethyl steht, $R_2$ und $R_3$ ausgewählt sind aus der Gruppe bestehend aus Piperidinyl, -N-[Alkyl($C_1$-$C_5$)]$_2$-pyrrolidino und Morpholino, dadurch gekennzeichnet, daß man ein 3,6-Bis(substituiertes)acridin der Formel

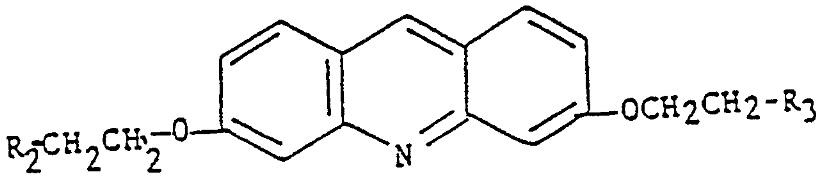

wobei $R_2$ und $R_3$ wie oben definiert sind, in konzentrierter Schwefelsäure mit p-Formaldehyd umsetzt; Ether zugibt, um Verbindungen der Formel

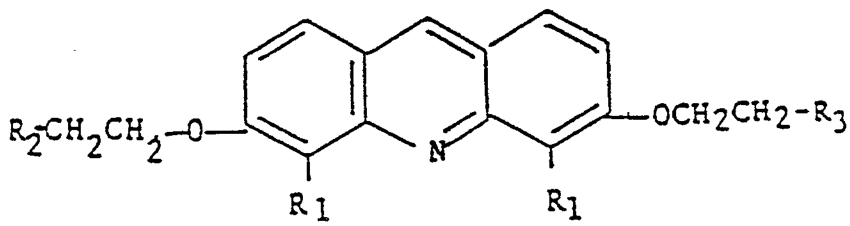

zu erhalten, wobei $R_2$ und $R_3$ wie oben beschrieben sind und $R_1$ für $-CH_2OH$ steht; diese Verbindungen in Dimethylsulfoxid auflöst; Triethylamin zusetzt; und mit Schwefeltrioxid-Pyridinkomplex umsetzt, um die Verbindungen der obigen Formel zu erhalten, bei denen $R_2$ und $R_3$ wie oben definiert sind und $R_1$ Formyl ist.

8. Verfahren zur Herstellung einer Verbindung der Formel

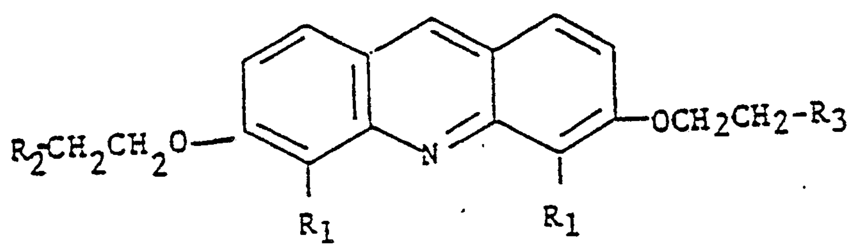

wobei $R_2$ und $R_3$ für Piperidinyl, -N-[Alkyl($C_1$-$C_5$)]$_2$, Pyrrolidin oder Morpholino stehen; und $R_1$ für Aminomethyl oder

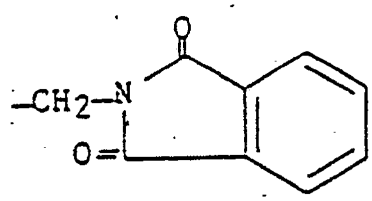

steht, dadurch gekennzeichnet, daß man ein 3,6-Bis(subsituiert)-acridin der Formel

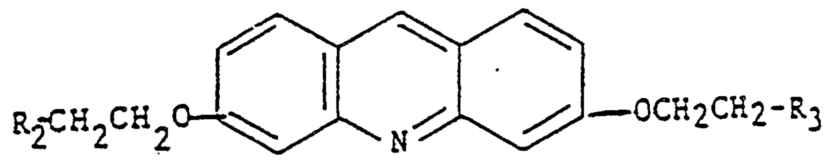

wobei $R_2$ und $R_3$ wie oben definiert sind, in konzentrierter Schwefelsäure mit Hydroxymethylphthalimid umsetzt, um Verbindungen der Formel

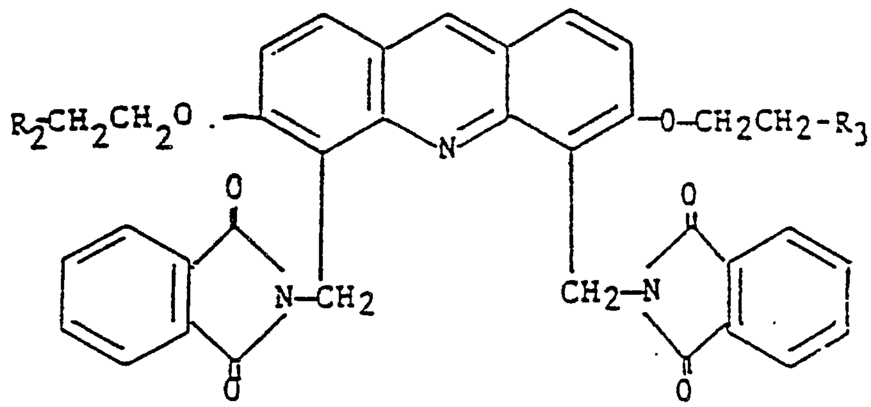

zu erhalten, diese Verbindungen mit Hydrazinhydrat in Ethanol am Rückfluß während etwa 2 bis etwa 4 h umsetzt, um Verbindungen der Formel

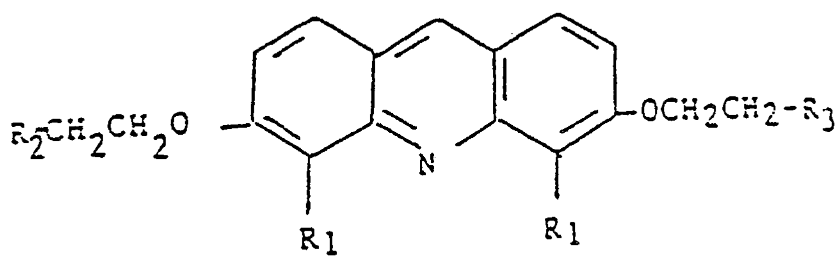

zu erhalten, wobei $R_2$ und $R_3$ wie oben definiert sind und $R_1$ Aminomethyl ist.

9. Verfahren zur Herstellung einer Verbindung der Formel

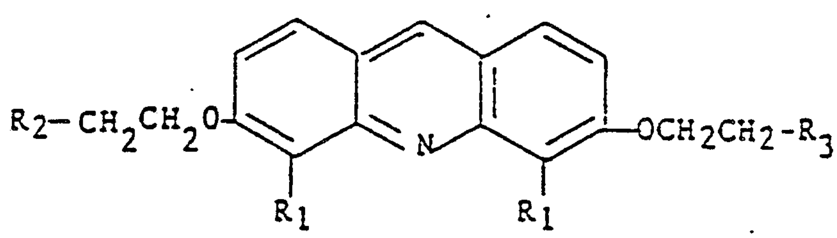

wobei $R_1$ ausgewählt ist aus der Gruppe bestehend aus Wasserstoff, Halogen, Hydroxymethyl, Formyl, -COOH und Alkyl($C_1$-$C_3$); $R_2$ und $R_3$ gleich oder verschieden sein können und ausgewählt sind aus der Gruppe bestehend aus

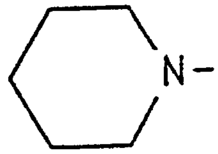
und
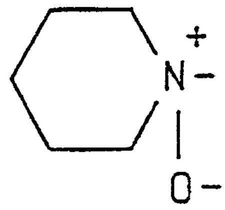

mit der Maßgabe, daß eines von $R_2$ und $R_3$ in der N-Oxidform vorliegen muß, dadurch gekennzeichnet, daß man eine Verbindung der Formel

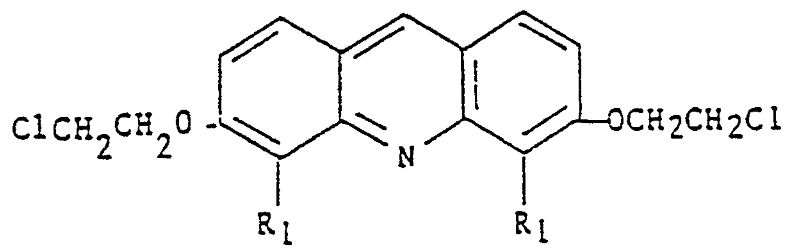

wobei $R_1$ wie oben definiert ist, mit Pyridin in einer Stahlbombe bei etwa 60 °C bis etwa 100 °C umsetzt, um ein 3,6-Bis-(2-piperidinoethoxy)acridin der Formel

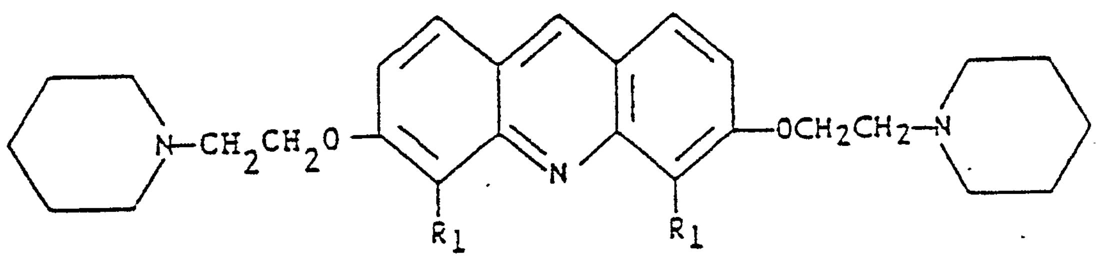

zu erhalten, diese Verbindungen in Methanol auflöst; mit einem Überschuß an 30%igem Wasserstoffperoxid behandelt; mit Platinkatalysator behandelt; und die Verbindungen durch trockene Säulenchromatographie reinigt, um in Abhängigkeit von der Reaktionszeit das Mono-N-oxid-oder das N,N-Dioxidderivat zu erhalten.

**10.** Verfahren zur Herstellung einer Verbindung, wie in Anspruch 1 definiert, der Formel

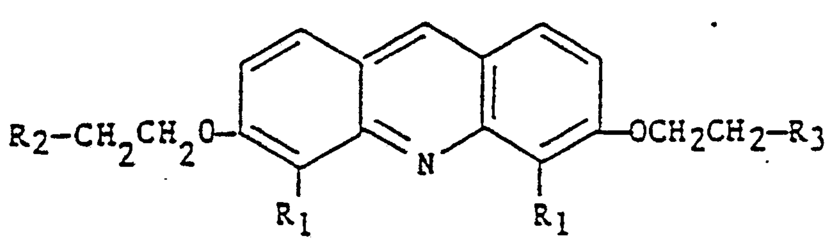

wobei $R_1$ ausgewählt ist aus der Gruppe bestehend aus Wasserstoff, Halogen, Hydroxymethyl, Formyl, -COOH und Alkyl-($C_1$-$C_3$); $R_2$ und $R_3$ gleich oder verschieden sein können und ausgewählt sind aus der Gruppe bestehend aus -N-[Alkyl($C_1$-$C_4$)$]_2$ und

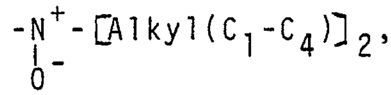

mit der Maßgabe, daß mindestens eines von $R_2$ und $R_3$ in der N-Oxidform vorliegen muß, dadurch gekennzeichnet, daß man eine Verbindung der Formel

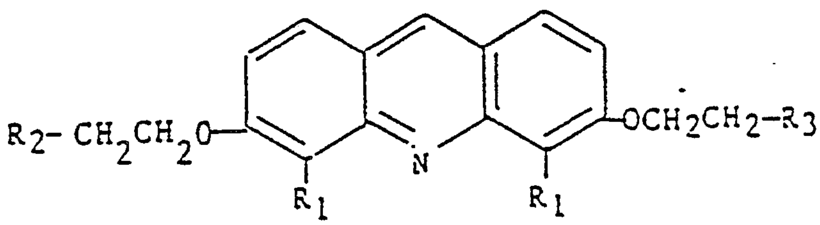

wobei $R_1$ wie oben definiert ist und $R_2$ und $R_3$ für -N-[Alkyl-($C_1$-$C_4$)]$_2$ stehen, in Methanol auflöst; mit einem Überschuß an 30%igem Wasserstoffperoxid behandelt; mit Ether behandelt; und durch Umkristallisation aus Acetonitril reinigt, um in Abhängigkeit von der Reaktionszeit das Mono-N-oxid- oder das N,N-Dioxidderivat zu erhalten.

**11.** Verfahren zur Herstellung einer Verbindung der Formel

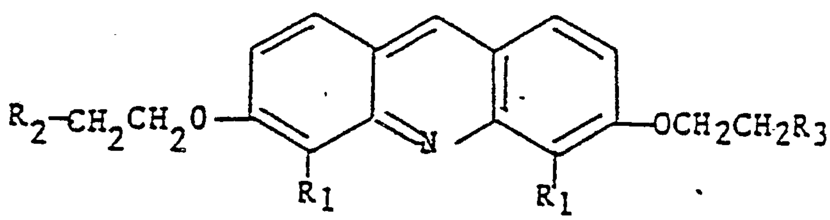

wobei $R_1$ ausgewählt ist aus Chlor und Methyl; $R_2$ und $R_3$ gleich oder verschieden sein können und für

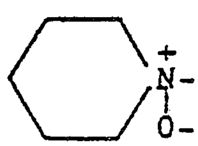 oder 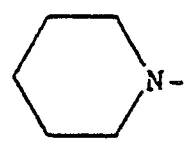

stehen, mit der Maßgabe, daß mindestens eines von $R_2$ und $R_3$ in der N-Oxidform vorliegen muß, dadurch gekennzeichnet, daß eine Verbindung der Formel

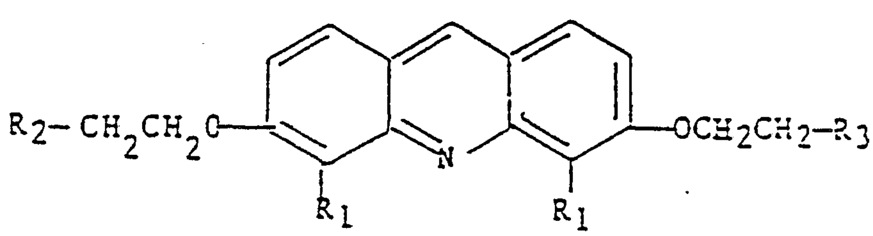

wobei $R_1$ wie oben definiert ist und $R_2$ und $R_3$ für

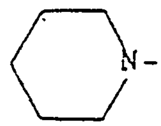

stehen, in n-Propanol auflöst; mit einem Überschuß an 30%igem Wasserstoffperoxid behandelt und aus Wasser und Acetonitril kristallisiert, um in Abhängigkeit von der Reaktionszeit das Mono-N-dioxid- oder das N,N-Dioxidderivat zu erhalten.